# EUROPÄISCHE PATENTSCHRIFT

(11) **EP 3 140 660 B1**
(45) Veröffentlichungstag und Bekanntmachung des Hinweises auf die Patenterteilung: **11.07.2018**
(21) Anmeldenummer: 15723455.0
(22) Anmeldetag: 06.05.2015
(51) Int. Cl.: G01N 33/72

(54) **ENZYMATISCHE BESTIMMUNG VON HBA1C**
ENZYMATIC DETERMINATION OF HBA1C
DÉTERMINATION ENZYMATIQUE DE HBA1C

(30) Priorität: 06.05.2014 DE 102014106301
(43) Veröffentlichungstag der Anmeldung: 15.03.2017
(73) Patentinhaber: DiaSys Diagnostic Systems GmbH, 65558 Holzheim (DE)
(72) Erfinder: GORKA, Günther, 65520 Bad Camberg (DE); WATAZU, Yoshifumi, Kobe 651-2276 (JP); METZMANN, Erwin, 35041 Marburg (DE); LEIN, Alexandra, 65582 Diez (DE); MÜLLER, Holger, 65582 Diez (DE); GRIMMLER, Matthias, 65604 Elz (DE)
(74) Vertreter: WSL Patentanwälte Partnerschaft mbB
(86) Internationale Anmeldenummer: PCT/EP2015/059970
(87) Internationale Veröffentlichungsnummer: WO 2015/169862

(56) Entgegenhaltungen:
- EP-A1- 2 357 228
- EP-A1- 2 813 854
- WO-A1-2013/118960
- US-A1- 2013 115 646
- STEFANO FERRI ET AL: "Review of Fructosyl Amino Acid Oxidase Engineering Research: A Glimpse into the Future of Hemoglobin A1c Biosensing Author Affiliations", JOURNAL OF DIABETES SCIENCE AND TECHNOLOGY, Bd. 3, Nr. 3, 1. Mai 2009 (2009-05-01), Seiten 585-592, XP055094382,

## Beschreibung

### Technisches Gebiet

Die vorliegende Erfindung betrifft ein Verfahren zur Bestimmung der Menge an glykiertem Hämoglobin (HbA1c) in einer Probe sowie ein Reagenz-Kit, das in einem Verfahren zur Bestimmung der Menge an glykiertem Hämoglobin (HbA1c) in einer Probe Anwendung finden kann.

### Hintergrund der Erfindung

Glykiertes Hämoglobin (HbA1c) ist nach der Definition der IFCC (International Federation of Clinical Chemistry and Laboratory Medicine) das stabile Produkt einer Kopplung von Glukose an das N-terminale Valin der Beta-Kette des Hämoglobins, und die Menge an HbA1c, bezogen auf die Menge des gesamten Hämoglobins (mmol HbA1c pro mol Gesamthämoglobin), ist repräsentativ für den mittleren Blutglukosespiegel der letzten acht Wochen vor der Blutprobenentnahme und wird daher auch als "Langzeit-Blutzucker" bezeichnet. Der HbA1c-Wert, der normalerweise im Bereich von 20 bis 42 mmol/mol liegen sollte, ist daher ein wichtiger Indikator für die Diagnose und Behandlung von Diabetes mellitus.

Bekannte Verfahren für die Bestimmung von HbA1c sind zum Beispiel Immunoassays, bspw. turbidimetrische Immunoassays (TIA) und die Hochleistungsflüssigchromatographie (HPLC). Seit einiger Zeit ist auch ein enzymatisches Verfahren verfügbar, bei dem eine Umsetzung des glykierten Hämoglobins mit einer Fructosylaminosäureoxidoreductase (FAOD) quantifiziert wird.

Bei der enzymatischen Untersuchung besteht - wie bei allen anderen HbA1c-Analyseverfahren im Übrigen auch - der erste Schritt darin, die Erythrocyten in der Blutprobe hämolytisch aufzuschließen, um das darin enthaltene HbA1c freizusetzen. Anschließend erfolgt das Inkontaktbringen des freigesetzten glykierten Hämoglobins mit einem proteolytischen Agens zur Erzeugung von glykierten Hämoglobinabbauprodukten. Diese proteolytisch erzeugten Abbauprodukte umfassen, die vom Amino-terminalen Ende der Beta-Kette von glykiertem Hämoglobin abgespaltenen Fructosyl-Valin (Fru-Val) und Fructosyl-Valin-Histidin (Fru-Val-His) oder gar längerkettige Fructosyl-Peptide. Die Fructosyl-Aminosäure bzw. das Fructosyl-Peptid wird durch die Aktivität des Enzyms Fructosylaminosäureoxidase (FAOX) bzw. des Enzyms Fructosylpeptidoxidase (FPOX) oxidiert, wobei ein Ergebnis dieses Oxidationsschritts die Erzeugung von Wasserstoffperoxid (H₂O₂) ist.

Die Menge an in dem vorgenannten Oxidationsschritt erzeugtem Wasserstoffperoxid korreliert mit der Menge an fructosylierter Aminosäure oder fructosyliertem Peptid. Demnach ist die in dieser Stufe erzeugte Menge an Wasserstoffperoxid ein Maß für die Menge an HbA1c in der Probe. Die Bestimmung der Menge an HbA1c kann daher letztlich dadurch erfolgen, dass man die Menge an Wasserstoffperoxid quantifiziert, beispielsweise anhand einer fotometrisch auszuwertenden Farbreaktion, die mit der Menge an Wasserstoffperoxid stöchiometrisch korreliert. Grundsätzlich ist jedoch auch jedes andere Analyseverfahren zur Quantifizierung der Menge an Wasserstoffperoxid in einer Probe entsprechend anwendbar.

Bei einem bestimmten Verfahren zur Quantifizierung von Wasserstoffperoxid wird ein reduzierter Leucofarbstoff mit Wasserstoffperoxid oxidiert. Dieses Verfahren bringt jedoch die Schwierigkeit mit sich, dass durch Autoxidation des Leucofarbstoff ein unspezifisches Leerwertsignal und eine Erhöhung des spektralen Untergrundes auftritt, das die präzise photometrische Messung des Analytsignals erschwert. Andererseits haben Leucofarbstoffe gegenüber anderen Farbstoffen den Vorteil, dass sie üblicherweise höhere molekulare Absorptionskoeffizienten aufweisen. Außerdem haben Leucofarbstoffe großteils so hohe Absorptionsmaxima, dass eine optische Beeinflussung durch Wechselwirkung mit Bestandteilen des Blutes, wie zum Beispiel Bilirubin und Hämoglobin, meist vernachlässigt werden kann.

### Aufgabe der Erfindung

Die Langzeit-Stabilität von chemischen und diagnostischen Reagenzien ist ein schwer zu lösendes Problem, insbesondere wenn die Reagenzien besonders hohen oder besonders niedrigen Temperaturen ausgesetzt sind und/oder signifikanten Temperaturveränderungen während des Transports, der Lagerung und/oder der Verarbeitung. Dies gilt in gleichem Maße wie für die oben besprochenen Leucofarbstoffe selbstverständlich auch für die in HbA1c-Tests eingesetzten Enzyme und anderen Reagenzien.

Gleichsam ist es jedoch auch von nicht unwesentlicher Bedeutung, die Stabilität des zu untersuchenden Ausgangsmaterials sowie der Zwischen- und Endprodukte der Reaktion sicherzustellen, um zuverlässig reproduzierbare Ergebnisse zu erzielen.

Die Erfinder der vorliegenden Anmeldung haben es sich daher zur Aufgabe gemacht, ein Verfahren zur Bestimmung der Menge an glykiertem Hämoglobin (HbA1c) in einer Probe und dabei anwendbare Reagenzien bereitzustellen, bei dem bzw. bei denen die reaktionswesentlichen chemischen Verbindungen in hinreichend stabiler Form vorliegen. Vorzugsweise soll mit dem erfindungsgemäßen Verfahren bzw. den erfindungsgemäßen Reagenzien außerdem gleichzeitig mit der Bestimmung der Menge an HbA1c auch eine Gesamthämoglobinbestimmung möglich sein.

### Beschreibung der Erfindung

Die oben beschriebene Aufgabe kann durch verschiedene weiter unten im Detail beschriebene Aspekte gelöst werden, denen üblicherweise gemein ist, dass sie aus einem Verfahren zur Bestimmung der Menge an HbA1c in einer Probe bestehen, bei dem man die folgenden Verfahrensschritte ausführt:
a) Hämolyse der Erythrocyten in der Probe zur Freisetzung des darin enthaltenen Hämoglobins, einschließlich HbA1c,
b) Inkontaktbringen des (in Verfahrensschritt a) freigesetzten) Hämoglobins, einschließlich HbA1c, mit einem proteolytisch wirkenden Agens zur Erzeugung von glykierten Hämoglobinabbauprodukten und
c) Bestimmung der Menge an HbA1c durch Quantifizierung der (in Verfahrensschritt b) erzeugten) glykierten Hämoglobinabbauprodukte.

Unter einer Probe wird im Zusammenhang mit der vorliegenden Erfindung jedes für Zwecke der Analyse aufbereitete Material verstanden, das einen zu analysierenden Anteil an Hämoglobin und/oder HbA1c enthält. In den meisten Fällen wird es sich bei der Probe um eine Probe von frischem Vollblut handeln. Von der vorliegenden Erfindung umfasst sind jedoch auch solche Proben, wie z.B. Blutkonserven, aufgereinigtes Blut, Vollblutlyophilisat, Erythrocytenkonzentrat, vorhämolysierte Blutproben, Hämoglobinstandardlösungen, HbA1c-Standardlösungen und Standardlösungen, die synthetische Hämoglobinabbauprodukte, wie z.B. synthetische HbA1c-Abbauprodukte, enthalten.

Soweit das HbA1c in der zu analysierenden Probe bereits frei vorliegt, ist der Verfahrensschritt a) entbehrlich. Im Falle von Standardlösungen, die synthetische Hämoglobin- bzw. HbA1c-abbauprodukte enthalten ist außerdem auch der Verfahrensschritt b) entbehrlich. Soweit das Hämoglobin bzw. das darin inhärent enthaltene HbA1c oder deren Abbauprodukte in der zu analysierenden Probe nicht bereits in Lösung vorliegen, werden diese vor oder während der Stufe a) in üblicherweise wässrige Lösung gebracht.

Die Hämolyse der Erythrocyten kann grundsätzlich mit allen mechanischen, chemischen oder osmotischen Hämolysemitteln oder -verfahren erfolgen, von denen der Fachmann weiß, dass sie zu einer vollständigen Hämolyse der Erythrocyten führen. Ein Mittel oder Verfahren gilt im Sinne der vorliegenden Erfindung dann als hämolytisch wirkend, wenn es zur Auflösung der Erythrozyten durch Zerstörung der Zellmembran und zum Übergang des in den Erythrocyten enthaltenen Hämoglobins in das umgebende Medium führt.

Beispiele für dem Fachmann bekannte hämolytisch wirkende Mittel oder Verfahren sind die Ultraschallbehandlung oder der Zusatz von hämolytischen Detergenzien oder von stark hypotonen Salzlösungen.

Bei der vorliegenden Erfindung kommen hämolytisch wirkende Detergenzien zum Einsatz. Diese können unter nicht-ionischen, anionischen, kationischen und zwitterionischen Detergenzien ausgewählt werden, wobei der Begriff Detergenz hier so verstanden wird, dass hierunter Substanzen fallen, die die Oberflächenspannung einer Flüssigkeit oder die Grenzflächenspannung zwischen zwei Phasen herabsetzen. Detergenzien sind organische Verbindungen, die aus einem unpolaren und einem polaren Teil aufgebaut sind, wobei der unpolare Teil zumeist eine Alkylgruppe oder eine Alkylbenzolgruppe ist und der polare Teil zumeist unter einer Alkohol-, Ether-, Alkohol-Ether-, Carboxyl-, Sulfonyl-, Sulfatyl- oder quaternären Ammoniumgruppe ausgewählt ist.

Bei bestimmten Ausführungsformen sind die eingesetzten hämolytisch wirkenden Detergenzien z.B. unter den Folgenden ausgewählt: hämolytische Polyethylenglykole (PEGs), hämolytische Glycoside und Ester, hämolytische Polyoxyethylenealkylether, hämolytische Polyoxyethylenealkylphenylether, hämolytisches Polyoxyethylenglycol, hämolytische Polyoxypropylenpolyoxyethylen-Triblockcopolymere (Poloxamere, Pluronics), n-Dodecyl-β-D-maltosid, n-Heptyl-β-D-thioglycosid, n-Octyl-β-D-thioglycosid, Saccharoselaureat, Saccharosecaprat, Saccharoselinolat, Saccharosepalmitat, Saccharosecholat und Derivaten der vorgenannten Verbindungen bzw. Mischungen davon.

Bei der vorliegenden Erfindung werden die hämolytisch wirkende Detergenzien in Form einer Hämolyselösung vorgehalten und eingesetzt. Vorzugsweise weist die Hämolyselösung einen Anteil an hämolytischem Detergenz im Bereich von 5 bis 50g/L Hämolyselösung auf. Bei bestimmten Ausführungsformen weist die Hämolyselösung einen Anteil an hämolytischem Detergenz im Bereich von 5 bis 30g/L Hämolyselösung oder im Bereich von 5 bis 15g/L Hämolyselösung auf.

Als proteolytisch wirkendes Agens kommen grundsätzlich alle dem Fachmann bekannten proteolytisch wirkenden Mittel in Betracht, wie z.B. Proteasen, wobei ein Mittel im Sinne der vorliegenden Erfindung dann proteolytisch wirkt, wenn es zur Spaltung von Proteinen durch Hydrolyse der Peptidbindungen führt.

Bei den Ausführungsformen, bei denen das proteolytische Agens eine Protease ist, kann diese grundsätzlich ausgewählt sein unter allen rekombinant aus Eukaryoten oder Prokaryoten hergestelllten oder endogen aus Organismen oder Organismembestandteilen gewonnenen Proteasen vom Serin-, Threonin-, Cystein-, Asparagin-, Metall- oder unbekanntem Typ, wie z.B. Akrosin, Aminopeptidase B, Bromelain, Calpain I, Carboxypeptidase A, Cathepsin A, Cathepsin B, Cathepsin D, Cathepsin E, Cathepsin K, Chymotrypsin, Collagenase, Dipeptidylpeptidase 4, Dispase, Elastase, Faktor IIa, Faktor Xa, Ficin, gpr-Endopeptidase, HIV-Protease, Kallikrein, MBTPS1, Bromelain, Papain, Pepsin, Plasmin, Prepilin Typ IV Peptidase, Prolyl-Oligopeptidase, Proteinase K, Proteasom, Renin, Sekretasen (Alpha-, Beta- und Gamma-Sekretase), Thermolysin (EC 3.4.24.27), Thrombin, Trypsin, Urokinase, Protease N aus *Bacillus sp.,* Protease P aus *Aspergillus* sp., Protease XIV aus *Streptomyces sp.* und Protease S aus *Bacillus stearothermophilus.*

Bei bestimmten Ausführungsformen der Erfindung kann es mit einem Vorteil verbunden sein, Proteasen einzusetzen, die spezifisch HbA1c spalten. Im Allgemeinen erfordert die vorliegende Erfindung jedoch keine Spezifität der Protease im Hinblick auf eine Differenzierung zwischen glykiertem und nicht-glykiertem Hämoglobin. Insofern können also auch solche Proteasen eingesetzt werden, die nicht spezifisch zwischen HbA1c und nicht-glykiertem Hämoglobin unterscheiden. Bei manchen Ausführungsformen kann es sogar explizit gewünscht sein, dass eine Protease eingesetzt wird, die nicht in entsprechender Weise spezifisch wirkt.

Bei manchen Aspekten der vorliegenden Erfindung ist es nicht erforderlich, dass die eingesetzte Protease zu bestimmten Abbauprodukten führen. Bei manchen Ausführungsformen der Erfindung wird jedoch gezielt eine Protease verwendet, deren proteolytische Aktivität zur Freisetzung von Fructosyl-Valin-Histidin oder Fructosyl-Valin vom Amino-terminalen Ende der Beta-Kette von glykiertem Hämoglobin führt.

Die Bestimmung der Menge an HbA1c kann grundsätzlich durch alle dem Fachmann bekannten Quantifizierungstechniken für die in Verfahrensschritt b) erzeugten glykierten Hämoglobinabbauprodukte erfolgen, wie z.B. durch eine HPLC-Analyse oder eine enzymatische Bestimmung, wie es weiter oben beschrieben wurde.

Neben dem oben beschriebenen Verfahren werden mit der vorliegenden Erfindung auch Reagenz-Kits zur Anwendung in einem Verfahren zur Bestimmung der Menge an HbA1c in einer Probe vorgeschlagen, die dadurch gekennzeichnet sind, dass sie wenigstens zwei verschiedene Lösungen in separaten Gebinden umfassen, wobei die wenigstens zwei verschiedenen Lösungen jeweils so zusammengesetzt sind, dass die verschiedenen weiter unten im Detail beschriebenen Aspekte der Erfindung realisiert werden.

### Aspekt 1 - Stabilisierung der Protease

Bei einer Ausführungsform der Erfindung werden zur Bestimmung der Menge an HbA1c in einer Probe - soweit erforderlich - die Verfahrensschritte a) bis c) ausgeführt.

Das in Verfahrensschritt b) eingesetzte proteolytisch wirkende Agens wird bei dieser Ausführungsform der Erfindung dadurch aktiviert, dass wenigstens zwei verschiedene Lösungen mit dem HbA1c in Kontakt gebracht werden, wobei die eine Lösung einen pH-Wert in dem Bereich von 1 bis 8 aufweist und
i) wenigstens eine Protease,
ii) pro 1000 kU/l an Protease jeweils 0,1 bis 2 mmol/l eines Chelators für zweiwertige Metallionen und
iii) 0,5 bis 10 mmol/l Ca²⁺ oder 0,5 bis 10 mmol/l Mg²⁺
enthält, wobei das molare Verhältnis Chelator:Ca²⁺ bzw. Chelator:Mg²⁺ in dem Bereich von 1:2 bis 1:20 liegt, und die andere Lösung 100 bis 5000 µmol/l eines zweiwertigen Metallions enthält, dass ausgewählt ist unter Fe²⁺, Mn²⁺, Co²⁺ und Zn²⁺.

Darüber hinaus wird für die hier beschriebene Ausführungsform ein Reagenz-Kit zur Anwendung in einem Verfahren zur Bestimmung der Menge an HbA1c in einer Probe vorgeschlagen, das dadurch gekennzeichnet ist, dass das Reagenz-Kit wenigstens zwei verschiedene Lösungen in separaten Gebinden umfasst, wobei die eine Lösung einen pH-Wert in dem Bereich von 1 bis 8 aufweist und die oben genannten Komponenten i) bis iii) enthält, wobei das molare Verhältnis Chelator:Ca²⁺ bzw. Chelator:Mg²⁺ in dem Bereich von 1:2 bis 1:20 liegt, und die andere Lösung 100 bis 5000 µmol/l eines zweiwertigen Metallions enthält, dass ausgewählt ist unter Fe²⁺, Mn²⁺, Co²⁺ und Zn²⁺.

Bei bestimmten Ausführungsformen der Erfindung beträgt der pH-Wert der die Protease enthaltenden Lösung wenigstens 4 und/oder höchstens 6. Bei anderen Ausführungsformen der Erfindung ist der pH-Wert der die Protease enthaltenden Lösung größer als 4 und/oder kleiner als 6. Bei spezifischen Ausführungsform der Erfindung liegt der pH-Wert der Lösung in dem Bereich von 4,5 bis 5,5 und bei sehr spezifischen Ausführungsformen in dem Bereich von 4,9 bis 5,1.

Die Aktivität der Metalloproteasen hängt von der Gegenwart zweiwertiger Metallionen wie Fe²⁺, Mn²⁺, Co²⁺ oder Zn²⁺ ab. Derzeit werden 54 Metalloprotease-Familien in 15 Clans unterschieden, wobei dem Clan MA mit 39 Familien eine hervorragende Bedeutung zukommt. Allein 19 dieser 39 Familien können den sogenannten neutralen Zink-Metalloproteasen zugeordnet werden. Die übrigen Metalloproteasen sind Mangan oder Kobalt-abhängig.

Zu dem Clan MA gehören u.a. die folgenden Metalloproteasen: Membran-Alanyl-Aminopeptidase, Peptidyl-Dipeptidase A, Thimet-Oligopeptidase, Oligopeptidase F (*Lactococcus*), Mycolysin, Immun-Inhibitor A (*Bacillus*), kleine neutrale Streptomyces-Protease, Leishmanolysin, Mikrobielle Collagenase, Collagenase colA, Matrix-Metallopeptidase 1, Serralysin, Fragilysin, Autolysin (*Chlamydomonas*), Astacin, Reprolysin, Neprilysin, IgA-spezifische Metalloendopeptidase, Tentoxilysin, Thermolysin, neutrale Staphylococcus-Protease, Carboxypeptidase Taq, lethaler Anthrax-Faktor, Deuterolysin, Fungalysin, Zellteilungsprotein ftsH, Cytophagalysin, Pappalysin 1, Ste24-Endopeptidase (*Saccharomyces*), HtpX-Endopeptidase (*E.coli*), Archaelysin, Peptidase blaR1, Peptidase prtB, Enhancin, Glycyl-Aminopeptidase (*Sphingomonas capsulata*), Peptidase IgA (*Clostridium ramosum*), Peptidase stcE (*E.coli*), Peptidyl-Asp-Metalloendopeptidase (*P.aeruginosa*) und ImmA-Peptidase.

Liegen Proteasen über einen längeren Zeitraum in ihrer aktiven Form vor, besteht die Gefahr, dass signifikante Anteile des Enzyms durch Eigenverdau zerstört werden. Insofern ist eine vorübergehende Inaktivierung der enzymatischen Aktivität von Proteasen häufig gewünscht, wenn vermieden werden soll, dass sich das Enzym selbst verdaut.

Das Problem des Eigenverdaues von Proteasen ist im Stand der Technik an sich bereits bekannt. Zur Lösung dieses Problems wurde im Stand der Technik beispielsweise für die Protease Thermolysin vorgeschlagen, Zink durch Chelatoren, insbesondere SH-Gruppen-haltige Reagenzien, oder durch einen einfachen Überschuss an EDTA aus dem Thermolysin zu entfernen.

Die Erfinder der vorliegenden Anmeldung haben jedoch realisiert, dass der Überschuss an Chelator einen negativen Einfluss auf die Stabilität der Protease haben kann, da der Chelator nicht nur - wie gewünscht - den für die katalytische Aktivität essentiellen Cofaktor aus der Protease entfernt, sondern zu einem signifikanten Anteil auch Ca²⁺ und/oder Mg²⁺, obwohl die Stabilität und strukturelle Integrität vieler Proteasen die Gegenwart von Mg²⁺ oder Ca²⁺ verlangt. Darüber hinaus haben die Erfinder festgestellt, dass die Bereitstellung der Protease in seiner inaktiven Form in einem Reagenzkompartiment bei einem spezifischen pH und die Reaktivierung der Protease mittels einem aus Fe²⁺, Mn²⁺, Co²⁺ und Zn²⁺ ausgewählten zweiwertigen Metallion, dass in einem zweiten Reagenzkompartiment bereitgestellt wird, die beste Form der Langzeitlagerung der Protease ohne signifikante Einbuße an Aktivität darstellt.

Da Calcium- oder Magnesiumionen für die Stabilität der Proteinstruktur der Protease von wesentlicher Bedeutung sind, bewirkt der Einsatz eines Überschusses an Chelator folglich eine Destabilisierung des Proteaseproteins. Die Erfinder haben jedoch herausgefunden, dass dieser Destabilisierung dadurch begegnet werden kann, dass ein erheblicher Überschuss an Ca²⁺ und/oder Mg²⁺ bereitgestellt wird, der bewirkt, dass der Einsatz des Chelators in der für die Deaktivierung der Protease erforderlichen Menge nicht mit einer Destabilisierung der Proteinstruktur der Protease einhergeht.

Entscheidend ist in diesem Zusammenhang vor allem das molare Verhältnis der eingesetzten Calcium- oder Magnesiumionen bezogen auf die eingesetzte Menge an Chelator, denn hierdurch wird gewährleistet, dass genügend Calcium- oder Magnesiumionen vorliegen, um die Proteinstruktur der Protease zu stabilisieren, ohne dabei die unter Fe²⁺, Mn²⁺, Co²⁺ und Zn²⁺ ausgewählten zweiwertigen Metalionen aus dem Chelat-Komplex mit dem Chelator zu verdrängen.

Beispiele für im Sinne der vorliegenden Ausführungsform der Erfindung geeignete Chelatoren für zweiwertige Metallionen sind Acetylaceton, Nitrilotriessigsäure (NTA), Ethylendiamin, Ethylendiamintetraacetat (EDTA), N-(2-Hydroxyethyl)-ethylendiamin-N,N,N'-triessigsäure Trinatriumsalz (HEDTA), Cyclohexandiamintetraessigsäure (CDTA), Ethylenglycol-bis(aminoethylether)-N,N,N',N'-tetraessigsäure (EGTA), 2-(2-Aminoethylamino)ethanol, Diethylentriamin, Iminodiacetat, Triethylentetramin, Triethylentetraminhexaessigsäure (TTHA), Triaminotriethylamin, Nitrilotriacetat, Bis(salicyliden)ethylendiamin, Ethylendiaminotriacetat, Ethylendiamintetraacetat, Diethylentriaminpentaacetat (DTPA), 1,4,7,10-Tetraazacyclododecan-1,4,7,10-tetraacetat, Oxalat, Tartrat, Citrat, Dimethylglyoxim, 8-Hydroxychinolin, 2,2'-Bipyridin, 1,10-Phenanthrolin, Dimercaptobernsteinsäure, 1,2-Bis(diphenylphosphino)ethan.

Der eingesetzte Chelator der unter Fe²⁺, Mn²⁺, Co²⁺ und Zn²⁺ ausgewählten zweiwertigen Metallionen bindet mit höherer Affinität an unter Fe²⁺, Mn²⁺, Co²⁺ und Zn²⁺ ausgewählten zweiwertigen Metallionen als an Calcium- oder Magnesiumionen. Vorzugsweise bindet der eingesetzte Chelator mit einer mindestens 10³-fach höheren Bindungsstärke an unter Fe²⁺, Mn²⁺, Co²⁺ und Zn²⁺ ausgewählten zweiwertigen Metallionen gegenüber Calcium- oder Magnesiumionen.

Die Chelator-Konzentration kann in Abhängigkeit von der Menge an Protease innerhalb des oben genannten Bereichs frei gewählt werden. Bei einer Ausführungsform enthält die Lösung pro 1000 kU/l an Protease 0,5 bis 1,5 mmol/l Chelator. Bei einer anderen Ausführungsform enthält die Lösung 0,9 bis 1,1 mmol/l Chelator pro 1000 kU/l an Protease.

Auch die Menge an Calcium- oder Magnesiumionen kann in den vorgenannten Konzentrationsbereichen frei gewählt werden. Bei einer Ausführungsform liegt die Calcium- bzw. Magnesiumionenkonzentration in dem Bereich von 3 bis 8 mmol/l. Bei einer anderen Ausführungsform liegt die Calcium- bzw. Magnesiumionenkonzentration in dem Bereich von 4 bis 7 mmol/l.

Die Metallionen können in der eingesetzten Reagenzienlösung in jeder geeigneten Salzform bereitgestellt werden, solange die gewählte Salzform die geforderte Menge an gelöstem Metallion im Ansatz zur Verfügung stellt, wie z.B. Chloride, Nitrate, Sulfate, Formiate und Acetate. Bei den Ausführungsformen, bei denen das Metallion Ca²⁺ oder Mg²⁺ im Ansatz zur Verfügung gestellt wird, ist das in der eingesetzten Reagenzienlösung hierfür vorliegende Calcium- oder Magnesiumsalz bei bestimmten Ausführungsformen also Calciumchlorid, -nitrat, -formiat -acetat bzw. das respektive Mg²⁺-Salz. Bei den Ausführungsformen, bei denen das zweiwertige Metallion unter Fe²⁺, Mn²⁺, Co²⁺ und Zn²⁺ ausgewählt ist, kann das in der eingesetzten Reagenzienlösung hierfür vorliegende Metallsalz also beispielsweise Eisenchlorid, Mangannitrat, Cobaltformiat oder Zinkacetat sein.

Das molare Verhältnis Chelator:Ca²⁺ bzw. Chelator:Mg²⁺ kann in dem Bereich von 1:2 bis 1:20 je nach Ausführungsform frei gewählt werden. Bei bestimmten Ausführungsformen der vorliegenden Erfindung liegt das molare Verhältnis Chelator:Ca²⁺ bzw. Chelator:Mg²⁺ in dem Bereich von 1:4 bis 1:10. Bei manchen Ausführungsformen liegt das Verhältnis Chelator:Ca²⁺ bzw. Chelator:Mg²⁺ in dem Bereich von 1:4 bis 1:8. Bei anderen Ausführungsform liegt das Verhältnis in dem Bereich von 1:5 bis 1:7.

Die Reaktivierung der in der zuvor beschriebenen Weise inaktivierten Protease erfolgt erfindungsgemäß durch den Zusatz einer Lösung mit 100 bis 5000 µmol/L des Metallions, das für die enzymatische Aktivität der jeweils eingesetzten Protease essentiell ist (Fe²⁺, Mn²⁺, Co²⁺ oder Zn²⁺). Die Erfinder der vorliegenden Anmeldung haben herausgefunden, dass diese spezifische Menge der jeweiligen zweiwertigen Metallionen genügt, um die für die Aktivierung der Protease erforderlichen Stellen in der Proteinstruktur der Protease trotz der gleichzeitig anwesenden signifikanten Mengen an Chelator und Calcium- oder Magnesiumionen wieder zu besetzen. Der Metallionengehalt der Aktivierungslösung kann in dem genannten Bereich je nach Ausführungsform frei gewählt werden. Bei bestimmten Ausführungsformen liegt der Metallionengehalt in dem Bereich von 200 - 2000 µmol/L oder im Bereich von 500 - 2000 µmol/L. Bei anderen Ausführungsformen liegt der Metallionengehalt in dem Bereich von 200 - 1000 µmol/L oder 300 - 1000 µmοl/L. Bei noch weiteren Ausführungsformen liegt der Metallionengehalt in dem Bereich von 200 bis 400 µmol/l.

Bei einer Ausführungsform der vorliegenden Erfindung ist die Lösung, die eines der unter Fe²⁺, Mn²⁺, Co²⁺ und Zn²⁺ ausgewählten zweiwertigen Metallionen enthält, eine Hämolyselösung, die in Verfahrensschritt a) zu der Probe zugegeben wird, wobei diese Hämolyselösung ein hämolytisches Detergenz enthält. Eine der Aufgaben der vorliegenden Erfindung ist es nämlich auch, möglichst viele der in der HbA1c-Bestimmung eingesetzten Reagenzien in möglichst wenige kombinierte Reagenzlösungen zusammenzufassen. In diesem Zusammenhang hat es sich herausgestellt, dass die unter Fe²⁺, Mn²⁺, Co²⁺ und Zn²⁺ ausgewählten zweiwertigen Metallionen, die zur späteren Aktivierung der Protease benötigt werden, durchaus auch in einer anderen Reagenzlösungen vorliegen können als in der, welche die Protease enthält. Beispielsweise liegen die unter Fe²⁺, Mn²⁺, Co²⁺ und Zn²⁺ ausgewählten zweiwertigen Metallionen bei manchen Ausführungsformen der Erfindung auch in der Lösung vor, mit der die FPOX oder die FAOX zugeführt wird (s.u. R1) oder auch in der Hämolyselösung, die gleich zu Beginn der HbA1c-Analyse eingesetzt wird.

Bei einer Ausführungsform der vorliegenden Erfindung werden die verschiedenen bei der HbA1c-Bestimmung eingesetzten Reagenzien in Form der folgenden Lösungen, die in separaten Gebinden vorgehalten werden, zusammengefasst:
- eine Hämolyselösung (H), enthaltend ein hämolytisches Detergenz und 100 bis 5000 µmol/L eines zweiwertigen Metallions, dass ausgewählt ist unter Fe²⁺, Mn²⁺, Co²⁺ und Zn²⁺,
- eine erste Reagenzienlösung (R1), enthaltend FPOX oder FAOX und Peroxidase, und
- eine zweite Reagenzienlösung (R2), zusätzlich enthaltend die Protease und pro 1000 kU/l an Protease jeweils 0,1 bis 2 mmol/l eines Chelators für zweiwertige Metallionen und 1 bis 10 mmol/l Ca²⁺ oder Mg²⁺ enthält, wobei das molare Verhältnis Chelator:Ca²⁺ bzw. Chelator:Mg²⁺ in dem Bereich von 1:2 bis 1:20 liegt, und einen Leucofarbstoff.

Das vorgenannte Reagenzien-Kit kann in einem Verfahren zur HbA1c-Bestimmung in der folgenden Weise eingesetzt werden. Zunächst erfolgt - soweit erforderlich - die Probenvorbereitung, bei der Vollblut mit der hämolysierenden Lösung versetzt wird. Zu dem daraus hervorgehenden Hämolysat wird dann die erste Reagenzlösung (R1) zugegeben, was dazu führt, dass die in der ersten Reagenzlösung enthaltene FPOX möglicherweise vorhandene endogene Fructosylpeptide bereits abbaut, nicht jedoch die für die HbA1c-Bestimmung relevanten endständigen fructosylierten Peptide, da diese noch nicht freigesetzt sind.

Nachdem die Reaktion im Wesentlichen abgeschlossen ist, erfolgt eine fotometrische Bestimmung des Gesamt-Hämoglobingehalts des vorinkubierten Hämolysats. Anschließend erfolgt die eigentliche Inkubation, bei der dem vorinkubierten Hämolysat die zweite Reagenzienlösung (R2) zugesetzt wird. Das darin enthaltene Protease-Apo-Enzym (z.B. Thermolysin-Apo-Enzym) wird durch die über die Hämolyselösung bereits im Ansatz enthaltenen zusätzlichen zweiwertigen Metallionen (z.B. Zinkionen) aktiviert und spaltet unter anderem N-terminales glykiertes Peptid von der Beta-Kette des Hämoglobins. Das abgespaltene glykierte Peptid wird dann von der FPOX umgesetzt, wobei bei der Spaltung des glykierten Peptids in Peptid und Glucoson Wasserstoffperoxid entsteht.

Die über die erste Reagenzienlösung (R1) bereits in den Ansatz eingebrachte Peroxidase bewirkt in Gegenwart des entstandenen Wasserstoffperoxids die Oxidation des Leucofarbstoffs hin zu dessen farbiger Oxidationsform. Die eigentliche HbA1c-Bestimmung kann dann durch fotometrische Messung beispielsweise sehr rasch (z.B. 10 bis 30 Sekunden, je nach Bauart des Messinstrumentes) nach dem Zusatz der zweiten Reagenzienlösung (R2), d.h. unmittelbar bevor die FPOX-vermittelte Reaktion einsetzt, erfolgen und noch einmal zu einem späteren Zeitpunkt (z.B. 2 bis 15 Minuten, je nach Bauart des Messinstrumentes) nach dem Zusatz von R2, d.h. nach Abschluss der Wasserstoffperoxid-bedingten Oxidation des Leucofarbstoffs. Die Bestimmung des HbA1c-Gehalts erfolgt letztlich anhand der Differenz der beiden Messungen und durch Bildung des Quotienten aus den Gehalten von HbA1c und dem zuvor bestimmten Gesamt-Hämoglobin. Die Messintervalle sind jedoch sehr stark abhängig von dem jeweils eingesetzten Analyzer, Photometer etc. Möglich wäre dementsprechend auch nur eine direkte Messung nach 2-15 Minuten

### Aspekt 2 - Stabilisierung des aufgefalteten Hämoglobins

Die Erfinder der vorliegenden Anmeldung haben sich die Aufgabe gestellt, das in einer Probe enthaltene Hämoglobin, einschließlich HbA1c, möglichst stark auffalten und in dieser aufgefalteten Form stabilisieren zu können, um beispielsweise einen möglichst effizienten Verdau des Hämoglobins durch eine Protease zu ermöglichen und das Häm in eine messbare, photometrisch stabile Form zu bringen.

Aus dem Stand der Technik ist bekannt, dass durch die Absenkung des pH-Wertes im Hämolysat Hämoglobin bis zu einem gewissen Grad aufgefaltet wird. Bei den herkömmlichen Verfahren zur Bestimmung der Menge an HbA1c erfolgt die Auffaltung bei einem pH-Wert von etwa 5. Eine stärkere Absenkung des pH-Wertes hat jedoch den Nachteil, dass das auf diese Weise behandelte Hämoglobin stark denaturiert und verklumpt und in dieser Form ausfällt, sodass es für den Verdau mit einer Protease nicht mehr in geeigneter Form zur Verfügung steht.

WO 2013/118960 A1 und EP 2 813 854 A1 offenbaren ein Verfahren zur Bestimmung der Menge an HbA1c in einer Probe, bei dem die Hämolyse der Erythrocyten mittels Zugabe von hämolytischen Agentien bewirkt wird. Bei dem Verfahren werden außerdem zwitterionische Detergentien zugesetzt. Nicht offenbart wird allerdings, dass die Hämolyselösung einen pH-Wert in dem Bereich von 1 bis 3 aufweist.

EP 2 357 228 A1 offenbart ebenfalls ein Verfahren zur Bestimmung der Menge an HbA1c in einer Probe, bei dem zwitterionische Detergentien zugesetzt werden. Nicht offenbart wird allerdings, dass die Hämolyselösung einen pH-Wert in dem Bereich von 1 bis 3 aufweist.

US 2013/115646 A1 offenbart ein Verfahren zur Bestimmung der Menge an HbA1c in einer Probe, bei dem Phosphoniumsalze zugesetzt werden. Nicht offenbart wird allerdings, dass die Hämolyselösung einen pH-Wert in dem Bereich von 1 bis 3 aufweist.

Die Erfinder der vorliegenden Anmeldung haben jedoch einen Weg gefunden, wie bei einem sehr niedrigen pH-Wert eine äußerst schnelle und effiziente Auffaltung und anschließende Stabilisierung des aufgefalteten Hämoglobins erreicht werden kann. Hierfür wird gemäß der vorliegenden Erfindung ein Verfahren zur Bestimmung der Menge an HbA1c in einer Probe vorgeschlagen, bei dem - soweit erforderlich - die Verfahrensschritte a) bis c) ausgeführt werden, wobei dieses Verfahren dadurch gekennzeichnet ist, dass die Hämolyse in Verfahrensschritt a) durch Zugeben einer Hämolyselösung (H) erfolgt, wobei diese Hämolyselösung einen pH-Wert in dem Bereich von 1 bis 3 aufweist und ein hämolytisches Detergenz und wenigstens einen Stabilisator enthält, wobei der wenigstens eine Stabilisator ein 2-(Methacryloyloxyethyl)-2'-(trimethyl-ammoniumethyl)phosphat-polymer oder-copolymer ist, wahlweise im Gemisch mit einem Phosphatidylcholin und/oder einem zwitterionischen Detergenz .

Unter einem Phosphatidylcholin wird hier eine Verbindung der allgemeinen Formel (I) 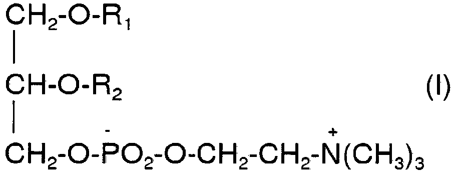 verstanden, wobei R₁ und R₂ ausgewählt sind unter vollständig gesättigten oder einfach oder mehrfach ungesättigten geradekettigen oder verzweigtkettigen Fettsäureresten. Bei den Ausführungsformen mit mehrfach ungesättigten Fettsäureresten sind diese vorzugsweise zweifach, dreifach oder vierfach ungesättigt, und unabhängig vom Sättigungsgrad sind bei bestimmten Ausführungsformen die Fettsäurereste ausgewählt unter solchen mit einer Kettenlänge in dem Bereich von C8 bis C22 oder solchen mit einer Kettenlänge in dem Bereich von C16 bis C22 (z.B. 1,2-Dioleoyl-sn-Glycero-3-Phosphocholin).

Unter einem 2-(Methacryloyloxyethyl)-2'-(trimethyl-ammoniumethyl)phosphatpolymer oder -copolymer wird hier ein Polymer oder Copolymer mit wiederkehrenden Einheiten der allgemeinen Formel (II) 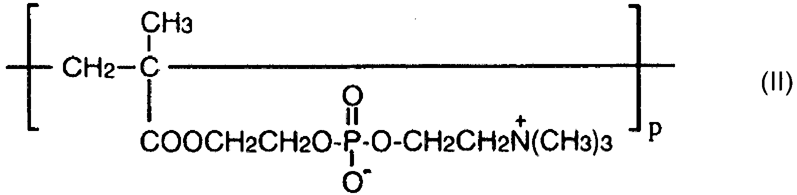 verstanden. Bei den Ausführungsformen, die als Copolymere vorliegen, umfassen diese neben den Einheiten mit der oben angegebenen allgemeinen Formel (II) weitere Methacrylat-Einheiten, die mit aliphatischen oder aromatischen Resten verestert sind.

Unter einem "zwitterionischen Detergenz" wird hier ein Detergenz verstanden, das zwei oder mehrere funktionelle Gruppen aufweist, von denen wenigstens eine positiv und wenigstens eine andere negativ geladen ist. Vorzugsweise hat das zwitterionische Detergenz exakt zwei funktionelle Gruppen mit entgegengesetzten Ladungen, sodass das Molekül insgesamt elektrisch neutral ist.

Vorzugsweise ist das zwitterionische Detergenz ausgewählt unter wenigstens einer chemischen Verbindung, die unter die folgende allgemeine Formel (III) 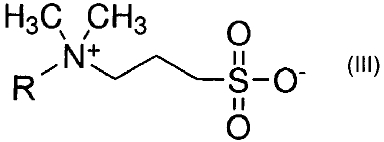 fällt, wobei R ausgewählt ist unter einem Alkylrest mit einer Kettenlänge in dem Bereich von C8 bis C20. Vorzugsweise ist R ausgewählt unter einem Alkylrest mit einer Kettenlänge in dem Bereich von C8 bis C16.

Der erfindungsgemäß eingesetzte Stabilisator kann eine einzige chemische Verbindung aus der Gruppe der 2-(Methacryloyloxyethyl)-2'-(trimethyl-ammoniumethyl)phosphatpolymere oder -copolymere (Verbindung, die unter die Formel (II) fällt sein oder ein Gemisch von 2 oder mehr chemischen Verbindungen der vorgenannten Art (Verbindung, die unter die Formel (II) fällt), wahlweise im Gemisch mit einem Phosphatidylcholin und/oder einem zwitterionischen Detergenz (Verbindung, die unter die Formel (I) und/oder die Formel (III) fällt). Vorzugsweise wird der Stabilisator mit einer Konzentration im Bereich von 0,1 bis 50g/L Hämolyselösung eingesetzt. Bei bestimmten Ausführungsformen wird der Stabilisator mit einer Konzentration im Bereich von 1 bis 30 g/L Hämolyselösung eingesetzt oder mit einer Konzentration im Bereich von 5 bis 15 g/L Hämolyselösung eingesetzt.

In den Fällen, in denen ein zwitterionisches Detergenz eingesetzt wird, das hämolytisch wirkt, oder aus einem oder mehreren hämolytisch wirkenden zwitterionischen Detergenzien besteht, sind verschiedene Varianten möglich:
a) Bei der Hämolyse wird zusätzlich ein hämolytisch wirkendes zwitterionisches Detergenz als Stabilisator eingesetzt, von dem sich alle weiteren hämolytischen Detergenzien, die verwendet werden, in ihrer chemischen Konstitution unterscheiden, sodass bei dieser Variante zwischen hämolytisch wirkendem Stabilisator und nicht stabilisierend wirkendem hämolytischem Detergenz zu differenzieren ist.
b) Bei der Hämolyse wird zusätzlich ein hämolytisch wirkendes zwitterionisches Detergenz als Stabilisator eingesetzt, von dem sich alle weiteren hämolytischen Detergenzien, die verwendet werden, dadurch unterscheiden, dass sie nicht-zwitterionisch sind sondern nicht-ionisch, einfach anionisch oder einfach kationisch, sodass auch bei dieser Variante zwischen hämolytisch wirkendem Stabilisator und nicht stabilisierend wirkendem hämolytischem Detergenz zu differenzieren ist.
c) Neben dem hämolytisch wirkenden zwitterionischen Detergenz wird bei der Hämolyse kein weiteres hämolytisches Detergenz verwendet, sodass bei dieser Variante der hämolytisch wirkende Stabilisator mit dem hämolytischen Detergenz gleichzusetzen ist.

Nach dem Zusatz der Hämolyselösung entspricht der pH-Wert des Hämolysates dem der Hämolyselösung. Dies wird erreicht durch eine entsprechende Pufferung der Hämolyselösung. Da gemäß dem vorliegenden Aspekt der Erfindung die Hämolyselösung einen pH-Wert in dem Bereich von 1 bis 3 aufweist, wird damit auch der pH-Wert der hämolysierten Probe (= Hämolysat) auf einen pH-Wert in dem Bereich von 1 bis 3 eingestellt. Der konstant niedrige pH des Hämolysats führt im Zusammenspiel mit den stabilisierenden Detergenzien dazu, dass das aufgefaltete Hämoglobin schnell und effizient abgebaut werden kann.

Außerdem wird ein Reagenz-Kit zur Anwendung in einem Verfahren zur Bestimmung der Menge an HbA1c in einer Probe vorgeschlagen, wobei das Reagenz-Kit wenigstens eine Hämolyselösung umfasst, die ein hämolytisch wirkendes Detergenz und 2-(Methacryloyloxyethyl)-2'-(trimethyl-ammoniumethyl)phosphatpolymer oder-copolymer enthält, wahlweise im Gemisch mit, einem Phosphatidylcholin und/oder einem zwitterionischen Detergenz oder Gemischen hiervon, und einen pH-Wert in dem Bereich von 1 bis 3 aufweist.

Die Absenkung des pH-Wertes in den Bereich von 1 bis 3 führt zu einer sehr schnellen und starken Auffaltung des Hämoglobins. Allerdings besteht, wie zuvor bereits erwähnt wurde, die Gefahr, dass das Hämoglobin denaturiert, verklumpt und ausfällt, wie dies beispielsweise auch durch Zusetzen von Trichloressigsäure bewirkt werden kann. Die Erfinder der vorliegenden Anmeldung haben jedoch herausgefunden, dass die Denaturierung, das Ausfallen und die Verklumpung des Hämoglobins vermieden werden kann, wenn die Hämolyselösung 2-(Methacryloyloxyethyl)-2'-(trimethyl-ammoniumethyl)phosphatpolymer oder -copolymer enthält, wahlweise im Gemisch mit einem Phosphatidylcholin und/oder eine, zwitterionischen Detergenz oder Gemischen hiervon.

Im Zusammenhang mit diesem Aspekt der Erfindung wird unter dem Begriff "Auffaltung" eine teilweise Auflösung der Sekundär- und Tertiärstruktur verstanden, die jedoch nicht zu einem Ausfallen des Hämoglobins führt. Das im Sinne der vorliegenden Erfindung aufgefaltete Hämoglobin verbleibt also in Lösung im Gegensatz zu denaturiertem Hämoglobin. Unter denaturiertem Hämoglobin wird im Sinne der vorliegenden Erfindung also solches Hämoglobin verstanden, dessen Proteinstruktur soweit aufgelöst wurde, dass das Protein ausfällt und verklumpt, wie dies beispielsweise durch Behandlung mit Trichloressigsäure erfolgt.

Die zuvor beschriebene starke Auffaltung des Hämoglobins bei gleichzeitiger Stabilisierung des aufgefalteten Produkts erfolgt überraschend schnell innerhalb von nur 5 bis 15 Sekunden, und dieser Effekt kann in Gegenwart von 2-(Methacryloyloxyethyl)-2'-(trimethyl-ammoniumethyl)phosphatpolymer oder -copolymer, wahlweise im Gemisch mit einem Phosphatidylcholin und/oder einem zwitterionischen Detergenz oder Gemischen hiervon in dem gesamten beanspruchten pH-Wertbereich von 1 bis 3 erreicht werden. Bei bestimmten Ausführungsformen der Erfindung liegt der pH-Wert in dem Bereich von 2 bis 3. Bei einer Ausführungsform liegt der pH-Wert der Hämolyselösung in dem Bereich von 2,4 bis 2,6.

### Aspekt 3 - Stabilisierung des Leucofarbstoffes

Eine Ausführungsform der vorliegenden Anmeldung betrifft insbesondere die Stabilisierung des Leucofarbstoffes bei den Verfahren zur Bestimmung der Menge an HbA1c in einer Probe, bei denen ein solcher Leucofarbstoff zum Einsatz kommt.

Unter einem Leucofarbstoff ist hier eine Substanz zu verstehen, die sich durch Reaktion mit mindestens einer oxidierenden Substanz und/oder einer Substanz mit Peroxidaseaktivität von einer farblosen Leuco-Ausgangsform in eine photometrisch messbare farbige Form umwandelt. Beispiele für im Sinne dieser Ausführungsform geeignete Leucofarbstoffe sind N,N,N',N',N",N"-Hexa(3-sulfopropyl)-4,4',4"-triaminotriphenylmethanhexanatriumsalz (TPM-PS), N-(Carboxymethylaminocarbonyl)-4,4'-bis(dimethylamino)-diphenylaminnatriumsalz (DA64), 10-(Carboxymethylaminocarbonyl)-3,7-bis(dimethylamino)-phenothiazinnatriumsalz (DA67), 2,2'-Azinobis(3-ethylbenzothiazolin-6-sulfonsäure (ABTS) und Triphenylamin-, Phenothiazin-, Phenoxazin-, Benzidin-, Triallylimidazol, o-Phenylendiaminderivate, Triphenylmethane und o-Tolidinderivate mit den vorgenannten Eigenschaften eines Leucofarbstoffes sowie Kombinationen von 4-Aminoantipyrin und einer Phenolverbindung oder einer N,N-disubstituierten Anilinverbindung und Kombinationen von 3-Methylbenzothiazolinonhydrozon (MBTH) und einer Anilinverbindung. Weitere Beispiele für im Sinne der vorliegenden Erfindung geeignete Leucofarbstoffe sind die in EP0038205, EP0124287 und EP0045220 beschriebenen Diphenylaminderivate.

Bei einer bestimmten Ausführungsform erfolgt die Stabilisierung des Leucofarbstoffes in dem Verfahren zur Bestimmung der Menge an HbA1c in einer Probe dadurch, dass bei dem Verfahren die Verfahrensschritte a) bis c) ausgeführt werden, und die Quantifizierung der glykierten Hämoglobinabbauprodukte in Verfahrensschritt c) durch deren Oxidation mit Hilfe von FPOX oder FAOX unter Erzeugung von Wasserstoffperoxid und durch Bestimmung der dabei entstehenden Menge an Wasserstoffperoxid erfolgt, wobei die Menge an Wasserstoffperoxid anhand der Farbreaktion eines Leucofarbstoffs in Gegenwart einer Peroxidase quantifiziert wird, und wobei der Leucofarbstoff in einer Lösung bereitgestellt wird, die zur Stabilisierung des Farbstoffes eine Verbindung der allgemeinen Formel (IV) enthält, 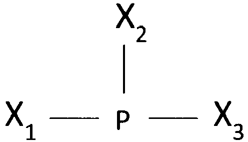 wobei P für ein Phosphoratom steht und wobei X₁, X₂ und X₃ unabhängig voneinander ausgewählt sind unter substituierten oder unsubstituierten gerade- oder verzweigtkettigen C₁-C₈-Alkylresten, substituierten oder unsubstituierten Cyclohexylresten und substituierten oder unsubstituierten Phenylresten.

Wie eingangs bereits erwähnt wurde, basiert das Prinzip der Leucofarbstoffe darauf, dass der Nachweis eines Stoffes darüber erfolgt, dass sich der Leucofarbstoff aufgrund der Anwesenheit des nachzuweisenden Stoffes von seiner farblosen Leuco-Ausgangsform in eine farbige Form umwandelt. Um sicherzustellen, dass diese Umwandlung nicht bereits vor der Durchführung der eigentlichen Nachweisreaktion erfolgt, ist es von Vorteil, wenn der Leucofarbstoff in seiner farblosen, reduzierten Form stabilisiert wird, ohne dass hierdurch die eigentliche Nachweisreaktion beeinträchtigt wird.

Die Erfinder der vorliegenden Anmeldung haben herausgefunden, dass durch den Zusatz von Verbindungen der allgemeinen Formel (IV) eine effektive Stabilisierung von Leucofarbstoffen erreicht werden kann, ohne dass hierdurch die eigentliche Nachweisreaktion (im vorliegenden Fall Nachweis von Wasserstoffperoxid in Gegenwart des Enzyms Peroxidase) gestört oder beeinträchtigt wird.

Beispiele für Verbindungen der allgemeinen Formel (IV) sind Tris(2-carboxyethyl)phosphatin (TCEP), Bis(p-sulfonatophenyl)phenylphosphin-dihydrat-dikaliumsalz, 1,3,5-Triaza-7-phosphin-adamantan, Tris(3-sulfonatophenyl)phosphinhydrat-natriumsalz, Tris(4,6-dimethyl-3-sulfonato-phenyl)phosphin-trinatriumsalzhydrat, Tris(hydroxymethyl)-phosphin, Di-t-butyl(3-sulfonatopro-pyl)phosphin, Diphenyl(m-sulfonatophenyl)phosphin-dihydrat-natriumsalz, [2-Dicyclohexylphos-phino)ethyl]-trimethylammoniumchlorid, ohne dass hierdurch eine Beschränkung dieser Ausführugsform der Erfindung auf diese lediglich beispielhaft genannten Verbindungen erfolgen soll.

Alternativ können zur Stabilisierung des Leucofarbstoffes auch Thio-Verbindungen, speziell einzelne oder mehrere Thioalkohole, Thioether, Thioketone oder Mischungen davon zugesetzt werden. Beispiele für solche Thio-Verbindungen sind Thiodiglycol, Thioäpfelsäure, Thionicotinamid, Thio-NAD und Gemische davon, ohne dass hierdurch irgendeine Beschränkung dieser Ausführugsform der Erfindung durch die Benennung dieser Beispiele erfolgen soll.

Die vorgenannten Thioverbindungen können entweder alleine oder in Kombination mit den weiter oben genannten Verbindungen der allgemeinen Formel (IV) eingesetzt werden. Bei bestimmten Ausführungsformen wird der Leucofarbstoff demnach in einer Lösung bereitgestellt, die zur Stabilisierung des Farbstoffes wenigstens eine Verbindung der allgemeinen Formel (IV), wie zum Beispiel TCEP, und wenigstens eine Thioverbindung, wie zum Beispiel Thiodiglycol, enthält. Die Kombination eines Stoffes nach Formel (IV) mit einer weiteren Thioverbindung aus der Gruppe der Thioketone, Thioether oder Thioakohole ist besonders geeignet, da unter Nutzung der jeweils individuellen Redoxpotentiale sowie der unterschiedlichen Stabilität und Reaktivität der genannten Thioverbindungen in wässrigen Lösungen eine optimale Stablisierung des Leucofarbstoffes über einen langen Zeitraum (bis 24 Monate) gewährleistet werden kann.

Die vorliegende Anmeldung betrifft dementsprechend auch ein Reagenz-Kit zur Anwendung in einem Verfahren zur Bestimmung der Menge an HbA1c in einer Probe, das dadurch gekennzeichnet ist, dass es wenigstens eine Reagenzienlösung umfasst, welche einen Leucofarbstoff enthält und zur Stabilisierung des Leucofarbstoffes wenigstens eine Verbindung der allgemeinen Formel (IV) und/oder wenigstens eine Thioverbindung.

Bei einer Ausführungsform liegt die Konzentration der Stabilisatorverbindung der allgemeinen Formel (IV) pro Mol an in der Reagenzlösung enthaltenem Leucofarbstoff in dem Bereich von 2,5 bis 20 mol. Bei bestimmten Ausführungsformen liegt die Konzentration in dem Bereich von 10 bis 20 mol. Bei speziellen Ausführungsformen liegt die Menge an Stabilisatorverbindung der allgemeinen Formel (IV) in dem Bereich von 5 bis 15 mol oder von 10 bis 15 mol pro Mol an Leucofarbstoff.

Bei den zuvor beschriebenen Verfahren zur Bestimmung der Menge an HbA1c in einer Probe werden häufig Reagenzien eingesetzt, die SH-Gruppen enthalten, wie zum Beispiel die vorgenannten Thioverbindungen. Solche SH-Gruppen haltigen Verbindungen können jedoch die eigentliche HbA1c-Nachweisreaktion stören und zwar an der Stelle, an der FPOX bzw. FAOX die fructosylierten Aminosäuren bzw. Peptide unter Bildung von Wasserstoffperoxid umsetzt und das daraus hervorgehende Wasserstoffperoxid quantifiziert wird. Insofern haben die Erfinder der vorliegenden Anmeldung gezeigt, dass bei der vorgenannten Nachweisreaktion störende SH-Gruppen möglichst blockiert werden müssen.

Es wird daher vorgeschlagen, zur Optimierung der eigentlichen HbA1c-Nachweisreaktion die diese Reaktion störenden SH-Gruppen durch Zusatz eines SH-Gruppen abfangenden Agens, wie z.B. N-Ethylmaleinimid (NEM), zu blockieren. Der Zusatz des SH-Gruppen abfangenden Agens sollte demnach spätestens unmittelbar vor Durchführung der HbA1c-Nachweisreaktion erfolgen.

Bei einer bestimmten Ausführungsform ist das SH-Gruppen abfangenden Agens, beispielsweise NEM, in der Hämolyselösung enthalten. Bei einer alternativen Ausführungsform ist das SH-Gruppen abfangenden Agens, beispielsweise NEM, in einer Reagenzlösung enthalten, die separat von der Reagenzlösung vorgehalten und zugesetzt wird, in der ein mit Thioalkoholen stabilisierter Leucofarbstoff vorliegt.

Das erfindungsgemäße Verfahren kann - sofern dies gewünscht ist - auch so durchgeführt werden, dass man während oder zwischen den Verfahrensschritten a) - c) eine Bestimmung der Gesamthämoglobinkonzentration durchführt. Dementsprechend schließt das Verfahren vorzugsweise einen Schritt zur Bestimmung der Gesamthämoglobinkonzentration ein, um die HbA1c-Konzentration unmittelbar als relative Menge, z.B. in mmol/mol angeben zu können.

### Spezielle Ausführungsformen der Reagenzien-Kits

Bei einer Ausführungsform der in der vorliegenden Anmeldung beschriebenen Erfindung wird ein Reagenz-Kit bereitgestellt, welches wenigstens die folgenden drei Lösungen in separaten Gebinden umfasst:
- eine Hämolyselösung (H), enthaltend ein hämolytisch wirkendes Detergenz,
- eine erste Reagenzienlösung (R1), enthaltend FPOX oder FAOX, und
- eine zweite Reagenzienlösung (R2), enthaltend eine Protease wobei:
   a) die Hämolyselösung (H) einen pH-Wert in dem Bereich von 1 bis 3 aufweist und 2-(Methacryloyloxyethyl)-2'-(trimethyl-ammoniumethyl)phosphatpolymer oder -copolymer, wahlweise im Gemisch mit einem Phosphatidylcholin und/oder einem zwitterionischen Detergenz, oder Gemischen hiervon, enthält und wahlweise
   b) die Hämolyselösung (H) 100 bis 5000 µmol/L eines zweiwertigen Metallions enthält, dass ausgewählt ist unter Fe²⁺, Mn²⁺, Co²⁺ und Zn²⁺, und die zweite Reagenzienlösung (R2) eine Protease enthält, deren proteolytische Aktivität zur Freisetzung von Fructosyl-Valin-Histidin oder Fructosyl-Valin vom Amino-terminalen Ende der Beta-Kette von glykiertem Hämoglobin führt, und pro 1000 kU/l an Protease jeweils 0,1 bis 2 mmol/l eines Chelators für zweiwertige Metallionen und 0,5 bis 10 mmol/l Ca²⁺ oder 0,5 bis 10 mmol/l Mg²⁺ enthält, wobei das molare Verhältnis Chelator:Ca²⁺ bzw. Chelator:Mg²⁺ in dem Bereich von 1:2 bis 1:20 liegt und wahlweise
   c) die zweite Reagenzienlösung (R2) einen Leucofarbstoff und einen Anteil einer Verbindung der allgemeinen Formel (IV) zur Stabilisierung dieses Leucofarbstoffes und/oder einen Anteil an wenigstens einer Thioverbindung zur Stabilisierung des Leucofarbstoffes enthält.

Bei den Ausführungsformen, bei denen die quantitative Bestimmung des HbA1c letztlich anhand des bei der Nachweisreaktion gebildeten Wasserstoffperoxids erfolgt, enthält wenigstens eine der vorgenannten Reagenzienlösungen eine Peroxidase und wenigstens eine andere der Reagenzienlösungen einen Leucofarbstoff. Bei diesen Ausführungsformen enthält wahlweise eine der Reagenzienlösungen außerdem ein SH-Gruppen abfangendes Agens, wie z.B. NEM, wobei das SH-Gruppen abfangende Agens in einer der Reagenzienlösungen enthalten ist, in der nicht der Leucofarbstoff enthalten ist.

Bei einer speziellen Ausführungsform der vorliegenden Erfindung besteht das Reagenz-Kit aus drei Reagenzienlösungen mit den folgenden Bestandteilen:
- Hämolyselösung, enthaltend ein hämolytisch wirkendes Detergenz, 2-(Methacry-loyloxyethyl)-2'-(trimethyl-ammoniumethyl)phosphatpolymer oder -copolymer, wahlweise im Gemisch mit einem Phosphatidylcholin und/oder einem zwitterionischen Detergenz, oder Gemische hiervon, ein Metallsalz zur Bereitstellung eines unter Fe²⁺, Mn²⁺, Co²⁺ und Zn²⁺ ausgewählten Metallions in der zuvor geforderten Menge und ein SH-Gruppen abfangendes Agens, wie z.B. NEM,
- eine erste Reagenzienlösung (R1), enthaltend FPOX bzw. FAOX und Peroxidase (POD), und
- eine zweite Reagenzienlösung (R2), enthaltend eine Protease, deren proteolytische Aktivität zur Freisetzung von Fructosyl-Valin-Histidin oder Fructosyl-Valin vom Amino-terminalen Ende der Beta-Kette von glykiertem Hämoglobin führt, ein Ca²⁺-Salz oder ein Mg²⁺-Salz und einen Chelator für zweiwertige Metallionen, jeweils in den zuvor geforderten Mengen, einen Leucofarbstoff und eine Verbindung der allgemeinen Formel (IV) zur Stabilisierung des Leucofarbstoffes und/oder eine Thioverbindung zur Stabilisierung des Leucofarbstoffes.

Für Zwecke der ursprünglichen Offenbarung wird darauf hingewiesen, dass sämtliche Merkmale, wie sie sich aus der vorliegenden Beschreibung, den Zeichnungen und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarer Merkmalskombinationen wird hier nur der Kürze und der Lesbarkeit der Beschreibung wegen verzichtet.

Des Weiteren wird darauf hingewiesen, dass es für den Fachmann selbstverständlich ist, dass die nachfolgenden Ausführungsbeispiele lediglich dazu dienen, die als Ausführungsbeispiele wiedergegebenen möglichen Ausführungsformen der vorliegenden Erfindung beispielhaft anzugeben. Der Fachmann wird daher ohne Weiteres verstehen, dass darüber hinaus auch alle anderen Ausführungsformen, die die in den Ansprüchen genannten erfindungsgemäßen Merkmale oder Merkmalskombinationen aufweisen, innerhalb des Schutzumfangs der Erfindung liegen. Auf die umfassende, explizite Darstellung sämtlicher denkbarer Ausführungsformen wird hier nur der Kürze und der Lesbarkeit der Beschreibung wegen verzichtet.

### Beispiele

### 1. Stabilisierung der Protease

### a) Stabilisierung der Protease, Analyse mittels SDS-Gelelektrophorese:

Die Protease Thermolysin weist bei Lagerung in flüssiger Form bzw. in Reagenzien über die Zeit bei 2-8°C oder höheren Temperaturen eine starke Tendenz auf, sich autoproteolytisch abzubauen. Dies steht der Nutzung der Protease für flüssig stabile Reagenzien mit gleichbleibender Qualitätsanforderung über längere Zeiträume im Weg. Die Protease Thermolysin ist Zn²⁺ abhängig, und die Erfinder konnten zeigen, dass die eine Proteaseinaktivierung mittels Chelator bei gleichzeitiger Ca²⁺-abhängiger struktureller Stabilisierung eine besser Nutzung von Thermolysin und verwandten Metalloproteasen für die klinisch-chemische Diagnostik ermöglicht.

Zum Nachweis der möglichen Stabilisierung einer Protease wurden in eine Reagenzgrundmatrix, in der die Protease Thermolysin enthalten war, jeweils unterschiedliche Mengen oder Verhältnisse von Chelator und Ca²⁺ eingebracht. Die dabei im Reagenz 2 eingesetzten Konzentrationen von EDTA und Kalzium sind in der folgenden Tabelle 1 angegeben.

**Tabelle 1:**

| Ansatz | [µmol/L] EDTA | [mmol/L] Ca²⁺ |
|---|---|---|
| E 411/13 A | 12 | 0,6 |
| E 411/13 B | 1000 | 6 |
| E 411/13 C | 12 | 6 |
| E 412/13 A | / | 6 |

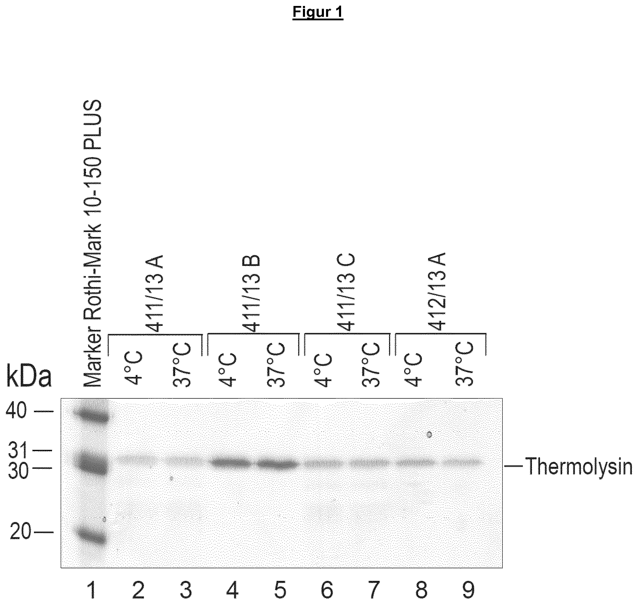

Die resultierenden Ansätze (siehe Tabelle oben) wurden aufgeteilt und bei 4°C bzw. bei 37°C über 7 Tage gelagert. Diesen Ansätzen wurde jeweils identische Probenmengen entnommen, mit SDS-PAGE Auftragspuffer versetzt und die Proteinkomposition denaturierend über SDS-PAGE aufgetrennt und per Coomassie-Anfärbung visualisiert (siehe Figur 1).

Evident sichtbar ist eine deutlich höhere Menge der Protease Thermolysin in den Spuren 4 und 5 im Vergleich zu den übrigen Spuren des Gels. Damit ist die Protease Thermolysin temperaturunabhängig in dieser Rezeptur gegen Selbstverdau deutlich besser geschützt als in den anderen Rezepturen. Der Vergleich der Ansätze mit der Nummer E411/13B zeigt, dass die Gegenwart von EDTA für den Schutz vor Selbstverdau essentiell ist. Der Vergleich der Ansätze mit der Nummer E411/13 A und E411/13C zu E411/13 B zeigt, dass eine gewisse Mindestmenge an EDTA erforderlich ist und dass auch die Kalziumionen in einer bestimmten Mindestmenge vorhanden sein müssen.

Darüber hinaus belegen die folgenden Daten die Thermolysin-stabilisierende Wirkung einer spezifischen Rezeptur bei einer Lagerung über einen längeren Zeitraum bei 4°C bzw. 37°C.

### b) Analyse der Stabilisierung der Protease im HbA1c-Testsystem

Die in Figur 1 auf proteinanalytischer Ebene gezeigte Stabilisierung der Protease wurde in einer HbA1c-Reagenzmatrix beispielhaft nachvollzogen. Dazu wurden in eine Reagenzgrundmatrix 2, mit jeweils gleicher Menge der Beispielprotease Thermolysin, verschiedene Kombinationen an Konzentrationen aus Kalzium-Ionen und EDTA zugefügt. Diese Ansätze wurden jeweils geteilt, direkt gemessen (frisch) bzw. Reagenz 2 wurde über 11 Tage bei 2-8°C (11d/2-8°C) bzw. Reagenz 2 wurde über 7 Tage bei 37°C und anschließend weitere 4 Tage bei 2-8°C gelagert (7d/37°C+4d/2-8°C).

Diese entsprechenden Reagenziengrundmatrices 2 wurden anschließend mit jeweils identischer Hämolyselösung und identischem Reagenz 1 an einem BM 6010c klinischen-chemisch Analyzersystem vermessen.

Zur Kalibration wurden DiaSys Kalibratoren TruCal HbA1c net (Artikel 1 3350) verwendet. Gemessen und bewertet wurde die HbA1c- Bestimmung der unterschiedlichen Reagenzmatrices 2 über die Zeit und bei unterschiedlichen Temperaturen gelagert, dargestellt in Tabelle 2. Δ mE entspricht dabei der Differenz Kalibrator Level 2 zum Kalibrator Level 1 in mE.

Die der Messung zu Grunde liegenden Grundrezepturen waren dabei wie folgt, wobei bei den einzelnen Versuchen einzelne Bestandteile (Calciumacetat/ETDA) in der Weise variiert wurden, wie dies in den weiter unten abgebildeten Tabellen angegeben ist:

### Hämolyselösung:

Glycin, 60 mol/l
N-Ethylmaleimid, 7,5 mmol/L
Triton X-100, 10g/l
2-(Methacryloyloxyethyl)-2'-(trimethyl-ammoniumethyl)phosphatpolymer, 50 g/L Zinkchlorid 1200 µmol/L
pH 2,5

### Reagenzlösung 1:

BIS-TRIS, 100 mmol/L
NaCl, 100 mmol/L
FPOX, größer 0,8 kU/L
POD, größer 50 kU/L
pH 7,2

### Reagenzlösung 2:

Natriumacetat, 20 mmol/L
NaCl, 100 mmol/L
Calciumacetat, Konzentration vgl. Tab. 2
TCEP, 0,1 mmol/L
DA-67, 0,08 mmol/L
Titriplex/EDTA, Konzentration vgl. Tab. 2
Protease (Thermolysin) 1000 U/ml
pH 5,0

Die der Messung zu Grunde liegende Applikation war dabei wie folgt:

| | |
|---|---|
| Reagenz 1: | 90 µL |
| Reagenz 2: | 30 µL |
| Hämolysierte Probe: | 15 µL |
| Hämolyseschritt: | 5 µL Probe + 100 µL Hämolyselösung |
| Wellenlänge (HbA1c-Best.): | 658 nm (main) / 805 nm (sub) |
| Messintervalle (Takte): | 22/23 - 41/42 |

**Tabelle 2:**

| **A) HbA1c Kalibrationssignale** | | | | | | | | | |
|---|---|---|---|---|---|---|---|---|---|
| **Betrachtung verschiedener R2 Variationen bei gleicher Hämolyselösung (ohne Zn²⁺)** | | | | | | | | | |
| **Lagerbedingung →** | **frisch** | | | **11d/2-8°C** | | | **7d/37°C+ 4d/2-8°C** | | |
| **TruCal HbA1c net →** | **Level 1** | **Level 2** | **ΔmE** | **Level 1** | **Level 2** | **ΔmE** | **Level 1** | **Level 2** | **ΔmE** |
| **R2 ↓** | | | | | | | | | |
| 6 mmol/L Ca | 6,9 | 11,3 | 4,5 | 6,8 | 11,8 | 5,0 | 6,8 | 10,1 | 3,4 |
| 12 µmol/L EDTA | 6,7 | 11,3 | | 7,0 | 11,9 | | 6,8 | 10,3 | |
| 6 mmol/L Ca | 6,6 | 11,1 | 4,5 | 6,8 | 11,3 | 4,7 | 6,8 | 10,5 | 3,4 |
| 1 mmol/L EDTA | 6,6 | 11,1 | | 6,7 | 11,5 | | 7,0 | 10,2 | |
| 0,5 mmol/L Ca | 6,5 | 11,1 | 4,7 | 6,5 | 11,1 | 4,6 | 6,3 | 10,3 | 4,0 |
| 12 µmol/L EDTA | 6,5 | 11,2 | | 6,5 | 11,1 | | 6,4 | 10,4 | |

| **B) HbA1c Kalibrationssignale** | | | | | | | | | |
|---|---|---|---|---|---|---|---|---|---|
| **Betrachtung verschiedener R2 Variationen bei gleicher Hämolyselösung (mit Zn²⁺)** | | | | | | | | | |
| **Lagerbedingung →** | **frisch** | | | **11d/2-8°C** | | | **7d/37°C+ 4d/2-8°C** | | |
| **TruCal HbA1c net →** | **Level 1** | **Level 2** | **ΔmE** | **Level 1** | **Level 2** | **ΔmE** | **Level 1** | **Level 2** | **ΔmE** |
| **R2 ↓** | | | | | | | | | |
| 6 mmol/L Ca | 16,7 | 32,9 | 16,1 | 17,1 | 34,1 | 16,9 | 13,4 | 25,8 | 12,5 |
| 12 µmol/L EDTA | 16,8 | 32,9 | | 17,0 | 33,8 | | 13,1 | 25,7 | |
| 6 mmol/L Ca | 29,8 | 70,2 | 40,1 | 29,2 | 69,8 | 41,1 | 19,2 | 52,5 | 32,8 |
| 1 mmol/L EDTA | 29,7 | 69,6 | | 29,0 | 70,5 | | 19,6 | 52,0 | |
| 0,5 mmol/L Ca | 30,1 | 71,2 | 41,6 | 21,2 | 46,8 | 25,7 | 12,4 | 26,4 | 14,1 |
| 12 µmol/L EDTA | 29,8 | 71,9 | | 20,8 | 46,7 | | 12,3 | 26,5 | |
| | | | | | | | | | |

| **C) Betrachtung Signalverlust über die Zeit verglichen zum jeweils frischen Wert** | | | | | | | | | |
|---|---|---|---|---|---|---|---|---|---|
| **Lagerbedingung →** | **frisch** | | | **11d/2-8°C** | | | **7d/37°C+ 4d/2-8°C** | | |
| **TruCal HbA1c net →** | **Level 1** | **Level 2** | **Δm E** | **Level 1** | **Level 2** | **ΔmE** | **Level 1** | **Level 2** | **AmE** |
| **R2↓** | | | | | | | | | |
| 6 mmol/L Ca | | | | 0,5 | 1,3 | 0,8 | -3,2 | -7,0 | -3,7 |
| 12 µmol/L EDTA | | | | 0,2 | 0,9 | | -3,6 | -7,2 | |
| 6 mmol/L Ca | | | | -0,6 | -0,4 | 0,9 | -10,6 | -17,7 | -7,3 |
| 1 mmol/L EDTA | | | | -0,7 | 0,9 | | -10,1 | -17,6 | |
| 0,5 mmol/L Ca | | | | -8,9 | -24,4 | 15,9 | -17,8 | -44,8 | -27,5 |
| 12 µmol/L EDTA | | | | -9,0 | -25,2 | | -17,5 | -45,4 | |

Aus Tabelle 2 A) ist abzuleiten, dass unabhängig von der Konzentration an Kalzium-Ionen und EDTA in Abwesenheit von Zink-Ionen keine HbA1c-Bestimmung möglich ist. Das unterschiedliche Signal für Level 1 und Level 2 bei verschiedenen Lagerbedingungen ist durch die unterschiedliche Konzentration an Hämoglobin der beiden Kalibratoren begründet.

Tabelle 2 B) zeigt hingegen Funktionalität von Thermolysin in Anwesenheit von Zink-Ionen bei unterschiedlichen Verhältnissen an Kalzium-Ionen und EDTA. In Tabelle 2 C) wird der ΔmE- Verlust der einzelnen Kalibratoren bei verschieden Lagerbedingungen gezeigt. Beide Ansätze mit 6 mmol/L Kalzium-Ionen zeigen einen deutlich geringeren Verlust an Signal im Gegensatz zu einem Ansatz mit 0,5 mmol/L Kalzium-Ionen.

Speziell bei der Lagerbedingung 7 Tage/37°C + 4 Tage/2-8°C ist der Unterschied der beiden Kalium-Konzentrationen bei gleicher Konzentration an EDTA signifikant. Die hier dargestellten Ergebnisse belegen eindrucksvoll die Lagerbarkeit einer inaktivierten aber strukturell stabilisierten Thermolysin in einer flüssigen Reagenzmatrix über diverse Temperaturen und über die Zeit sowie dessen Reaktivierbarkeit.

### 2. Stabilisierung des aufgefalteten Hämoglobins

Für die akkurate Messung von Hb bzw. HbA1c ist eine vorangehende effiziente Auffaltung von Hämoglobin wie auch die Stabilisierung dieser aufgefalteten Form von essentieller Bedeutung. Für Zwecke der Stabilisierung des aufgefalteten Hämoglobins bei der Hämolyse durch ein hämolytisches Detergenz wurde der Hämolyselösunggrundmatrix in dem folgenden Versuch exemplarisch z.B. 2-(Methacryloyloxyethyl)-2'-(trimethyl-ammoniumethyl)phosphatpolymer zugegeben und hierbei ein pH-Wert in dem Bereich 1 bis 3 eingestellt.

Diese verschiedenen Hämolyselösungs-Variationen mit bzw. ohne Zusatz wurde mit jeweils identischem Reagenz 1 und Reagenz 2 an einem klinisch-chemischen Analyzer Hitachi 912 vermessen (Reagezrezepturen wurden wie unter 1b) angegeben, entsprechend wie in den Tabellen beschrieben modifiziert eingesetzt) Um die Stabilität des Hämolysates zu bewerten, wurde das Hämolysat manuell hergestellt (50 µL Vollblut-Probe + 1000 µL je Hämolyselösung (ohne bzw. mit Zusatz an 2-(Methacryloyloxyethyl)-2'-(trimethyl-ammoniumethyl)phosphatpolymer) und direkt vermessen. Anschließend wurde das Hämolysat offen im Gerät bei 15 - 25°C gelagert und zu verschiedenen Zeitpunkt bis maximal 240 Minuten nach Herstellung der Hämolysate vermessen. Dabei wurde das Signal der HbA1c Bestimmung über einen Zeitraum von 240 min am Analyzer gemessen und jeweils in Relation zum frisch ermittelten HbA1c-Wert gesetzt. Folgende Applikation lag dabei der Messung am Hitachi 912 zugrunde:

| | |
|---|---|
| Reagenz 1: | 240 µL |
| Reagenz 3: | 80 µL |
| Hämolysierte Probe: | 40 µL |
| Wellenlänge (HbA1c-Best.): | 660 nm (main) / 800 nm (sub) |
| Messtakte: | 18 - 31 |

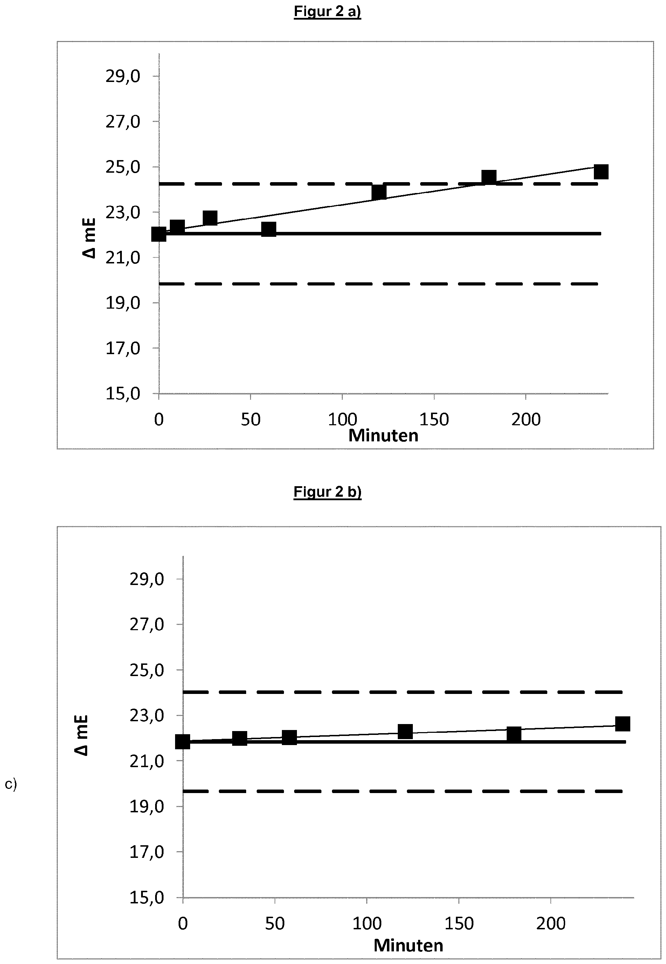

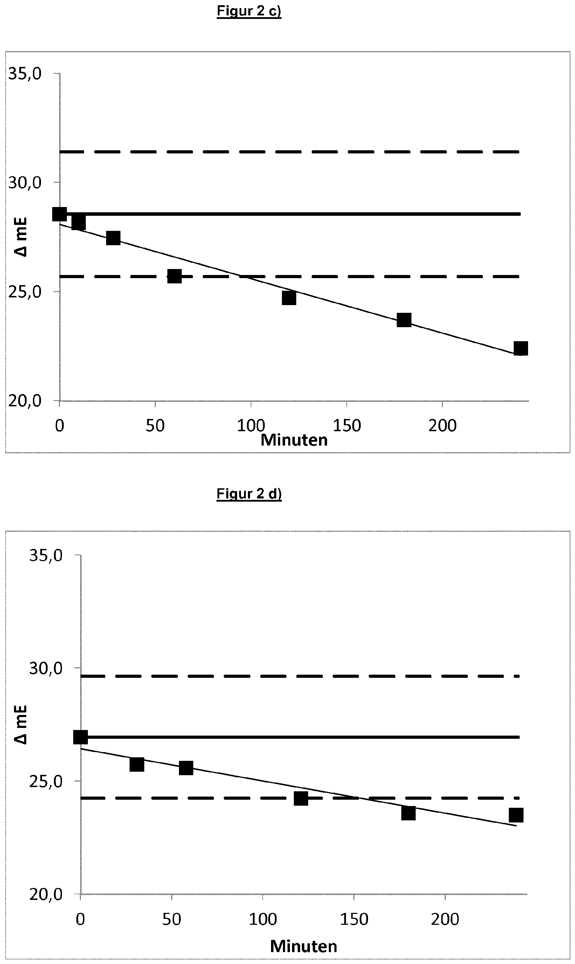

Die in Figur 2a-d dargestellten Ergebnisse zeigen eine deutliche Stabilitätszunahme für verschiedene Probenmaterialen mit 2-(Methacryloyloxyethyl)-2'-(trimethyl-ammoniumethyl)phosphatpoly-mer (mit Zusatz) im Vergleich zu Proben ohne 2-(Methacryloyloxyethyl)-2'-(trimethyl-ammoniume-thyl)phosphatpolymer (ohne Zusatz), Figur 2a-d. Im Einzelnen sind in der Figur 2 die Ergebnisse der folgenden Ansätze dargestellt:

| | |
|---|---|
| Figur a) | TruCal HbA1c Level 1 ohne Zusatz |
| Figur b) | TruCal HbA1c Level 1 mit Zusatz |
| Figur c) | low HbA1c, medium Hemoglobin ohne Zusatz |
| Figur d) | low HbA1c, medium Hemoglobin mit Zusatz |

Anhand der dargestellten Ergebnisse ist evident gezeigt worden, dass durch Zugabe von 2-(Methacryloyloxyethyl)-2'-(trimethyl-ammoniumethyl)phosphatpolymer in die Hämolyselösung unter den beschriebenen Bedingungen die Stabilisierung des Hämoglobinmoleküls deutlich verbessert wird, angegeben durch 10% Grenzen (gestrichelte Linie +/- 10%) zur jeweils frisch gemessenen HbA1c-Bestimmung.

### 3. Stabilisierung des Leucofarbstoffes mit Phosphinen

Um die Stabilität des Leucofarbstoffes in einer Reagenzmatrix zu verbessern wurden verschiedene wasserlösliche, stabile Substanzen auf ihre Leucofarbstoff-stabilisierende Wirkung hin geprüft. Als negativ-Referenz diente hierbei eine Reagenzrezeptur ohne Stabilisatorzusatz sowie als Kontrollsubstanz 1,3,5-Triaza-7-phosphaadamantan, da diese Substanz nicht den strukturellen Voraussetzungen entspricht.

Es wurde folgende exemplarische Reagenzpräparation hergestellt:
20mM BisTris Puffer (Bis(2-hydroxyethyl)amino-tris(hydroxymethyl)methan)
20 mM Na-Acetat
100 mM NaCl
6mM Ca-acetat
15g/L Triton X 405
0,08 mM DA67 (Leucofarbstoff)
1000 U/ml Thermolysin
1 mM EDTA

Zu der genannten Reagenzgrundmatrix ohne Stabilisator wurde nun jeweils ein unterschiedlicher Stabilisator in jeweils identischer Konzentration zugesetzt. Ein Aliquot ohne Stabilisatorsubstanz diente dabei als Referenz.

Die Eigenabsorption des jeweiligen Reagenzansatzes wurde bei 660 nm wurde jeweils direkt nach dem Ansetzten (frisch) und jeweils nach einer Lagerung von 4 Tagen bei 37°C am Photometer gemessen (Belastungsmessung). Die Zunahme der Extinktion 660nm über die Zeit ist dabei ein Maß für die Überführung der Leucoform des Farbstoffes in seine farbige Variante und somit ein Maß für die oxidative Destabilisierung der Leucoform.

Unter den oben angegebenen Bedingungen wurden die in Tabelle 3 gezeigten Ergebnisse erzielt.

**Tabelle 3: Eigenfärbung R2 bei 660 nm in mE**

| | **Tag 0** | **Tag 4** |
|---|---|---|
| ohne Stabilisator | 17,7 | 455 |
| TCEP | 15,7 | 37,9 |
| Bis(p-sulfonatophenyl)phenylphosphin-Dihydrat-Dikaliumsalz | 19,2 | 164 |
| 1,3,5-Triaza-7-phosphaadamantan | 20,7 | 391 |
| Tris(3-sulfonatophenyl)phosphinhydrat-Natriumsalz (<5% Oxid) | 18,7 | 251 |
| Tris(hydroxymethyl)phosphin | 19,9 | 239 |
| Diphenyl(m-sulfonatophenyl)phosphin-Dihydrat-Natriumsalz | 18,2 | 70 |
| [2-Dicyclohexylphosphino)ethyl]-trimethylammoniumchlorid | 18,6 | 307 |

### 4. Stabilisierung des Leucofarbstoffes mit Thioverbindungen

Es wurden mehrere Thioverbindungen auf ihre Farbstoff-stabilisierende Wirkung hin geprüft. Hierfür wurde die Eigenfärbung einer den Leucofarbstoff DA 67 enthaltenden Reagenzienlösung (Matrix aus: NaCl, Ca-Acetat, Triton X 405 + Thermolysin, siehe dazu Rezeptur 3.) nach Lagerung bei 2-8°C und 37°C untersucht. Dabei wurden für die Lagerung bei 2-8°C die in Tabelle 4 angegebenen Ergebnisse erzielt.

**Tabelle 4: Eigenfärbung R2 bei 660 nm in mE**

| | **Tag 0** | **Tag 2** | **Tag 8** | **Tag 10** |
|---|---|---|---|---|
| ohne Zusatz | 50,1 | 136 | 344 | 463 |
| 0,1 mM TCEP | 20,4 | 23,4 | 48,1 | 106 |
| 0,2 mM TCEP | 14,2 | 18,7 | 41,6 | 97,4 |
| 2 mM Thionicotinamid | 50,6 | 73,9 | 133,5 | 205 |
| 2 mM Thionicotinamid + 0,2mM TCEP | 9,6 | 17 | 41,5 | 91,8 |
| 4 mM Thionicotinamid | 55,7 | 79,9 | 122 | 209 |
| 2 mM Thio-NAD | 74,3 | 138 | 215 | 292 |
| 4 mM Thio-NAD | 97,9 | 139 | 203 | 274 |
| 4 mM Thionicotinamid + 0,1mM TCEP | 26,7 | 36,3 | 55,5 | 106 |
| 2 mM Thio-NAD+0,2mM TCEP | 11,1 | 34,6 | 56,6 | 105 |
| 4 mM Thio-NAD+0,1mM TCEP | 21,3 | 46,3 | 68,3 | 128 |

Für die Lagerung bei 37°C wurden die in Tabelle 5 angegebenen Ergebnisse erzielt.

**Tabelle 5: Eigenfärbung R2 bei 660 nm in mE**

| | **Tag 0** | **Tag 2** | **Tag 8** | **Tag 10** |
|---|---|---|---|---|
| ohne Zusatz | 50,1 | 610 | 2220 | 2513 |
| 0,1 mM TCEP | 20,4 | 23,4 | 66,2 | 646 |
| 0,2 mM TCEP | 14,2 | 18,7 | 31,9 | 295 |
| 2 mM Thionicotinamid | 50,6 | 73,9 | 233 | 671 |
| 2 mM Thionicotinamid + 0,2mM TCEP | 9,6 | 17 | 41,5 | 29,7 |
| 4 mM Thionicotinamid | 55,7 | 79,9 | 215 | 704 |
| 2 mM Thio-NAD | 74,3 | 138 | 366 | 1174 |
| 4 mM Thio-NAD | 97,9 | 139 | 305 | 893 |
| 4 mM Thionicotinamid + 0,1 mM TCEP | 26,7 | 36,3 | 55,5 | 48,8 |
| 2 mM Thio-NAD+0,2mM TCEP | 11,1 | 34,6 | 56,6 | 51,5 |
| 4 mM Thio-NAD+0,1mM TCEP | 21,3 | 46,3 | 68,3 | 109 |

### 5. Stabilisierung des Leucofarbstoffes mit TCEP in Kombination mit Thioverbindungen

Es wurden mehrere Thioverbindungen auf ihre Farbstoff-stabilisierende Wirkung bei Verwendung in Kombination mit TCEP geprüft. Hierfür wurde die Eigenfärbung einer den Leucofarbstoff DA 67 enthaltenden Reagenzienlösung (Matrix aus: Puffer, NaCl, Ca-Acetat, Triton X 405 + Thermolysin) nach Lagerung bei 2-8°C und 37°C untersucht. Dabei wurden für die Lagerung bei 2-8°C die in Tabellen 6a und 6b angegebenen Ergebnisse erzielt.

**Tabelle 6a: Eigenfärbung R2 bei 660 nm in mE**

| | **Tag 0** | **Tag 2** | **Tag 8** | **Tag 10** |
|---|---|---|---|---|
| ohne Zusatz | 128 | 564 | 940 | 1124 |
| 2 mM S-NAD | 228 | 437 | 657 | 832 |
| 2 mM Thionicotinamid | 73 | 189 | 268 | 319 |
| 2mM NAD | 62 | 619 | 1248 | 1508 |
| 2 mM Thioharnstoff | 85 | 514 | 844 | 1056 |
| 1% 2,2 Thiodiglycol | 96 | 211 | 299 | 357 |
| 0,1 mM TCEP | 89 | 111 | 129 | 266 |
| 2,2 Thiodiglycol+TCEP 0,1mM | 83 | 92 | 104 | 170 |

**Tabelle 6b: Eigenfärbung R2 bei 660 nm in mE (separater Ansatz)**

| | **Tag 0** | **Tag 2** | **Tag 8** | **Tag 10** |
|---|---|---|---|---|
| ohne Zusatz | 115 | 323 | 848 | 1154 |
| 2 mM Acetyl-NAD | 169 | 1097 | 1869 | 2037 |
| 2 mM Thioäpfelsäure | 91 | 114 | 158 | 197 |

Für die Lagerung bei 37°C wurden die in Tabellen 7a und 7b angegebenen Ergebnisse erzielt.

**Tabelle 7a: Eigenfärbung R2 bei 660 nm in mE**

| | **Tag 0** | **Tag 2** | **Tag 8** | **Tag 10** |
|---|---|---|---|---|
| ohne Zusatz | 128 | 957 | 2583 | 2821 |
| 2 mM S-NAD | 228 | 437 | 662 | 1591 |
| 2 mM Thionicotinamid | 73 | 189 | 292 | 862 |
| 2mM NAD | 62 | 1220 | 2717 | 2940 |
| 2 mM Thioharnstoff | 85 | 514 | 594 | 2152 |
| 1% 2,2 Thiodiglycol | 96 | 211 | 494 | 1730 |
| 0,1 mM TCEP | 89 | 111 | 219 | 1350 |
| 2,2 Thiodiglycol+TCEP 0,1mM | 83 | 92 | 104 | 272 |

**Tabelle 7b: Eigenfärbung R2 bei 660 nm in mE (separater Ansatz)**

| | **Tag 0** | **Tag 2** | **Tag 8** | **Tag 10** |
|---|---|---|---|---|
| ohne Zusatz | 115 | 1183 | 2714 | 2885 |
| 2 mM Acetyl-NAD | 169 | 1097 | 2024 | 3209 |
| 2 mM Thioäpfelsäure | 91 | 114 | 188 | 341 |

### 6. Signalstabilisierende Wirkung von Thiodiglycol alleine oder in Verbindung mit TCEP

Es wurden die HbA1c-Kalibrationssignale in Gegenwart von Thiodiglycol alleine oder in Verbindung mit TCEP untersucht.

Es wurde eine Reagenz 2-Grundmatrix (Reagezrezepturen wurden wie unter 1b) angegeben, entsprechend wie in den Tabellen beschrieben modifiziert eingesetzt) mit 10 g/L β-Thiodiglycol versetzt und Teile dieser Grundmatrix mit jeweils unterschiedlichen Konzentration an TCEP versetzt.

Anschließend wurde diese Reagenz2-Variationen mit jeweils einer identischen Hämolyselösung und Reagenz 1 an einem BM 6010 c vermessen. Dazu wurden die DiaSys Kalibratoren TruCal HbA1c net (Artikel 1 3350) verwendet. Gemessen und bewertet wurden dabei jeweils die HbA1c-Bestimmung.

Die der Messung zu Grunde liegende Applikation für den BM 6010c war dabei wie folgt:

| | |
|---|---|
| Reagenz 1: | 90 µL |
| Reagenz 3: | 30 µL |
| Hämolysierte Probe: | 15 µL |
| Hämolyseschritt: | 5 µL Probe + 100 µL Hämolyselösung |
| Wellenlänge (HbA1c-Best.): | 658 nm (main) / 805 nm (sub) |
| Messtakte: | 22/23 - 41/42 |

Dabei wurden die in Tabelle 8 angegebenen Ergebnisse erzielt.

**Tabelle 8:**

| **HbA1c Kalibrationssignale** | | | | | | | | | |
|---|---|---|---|---|---|---|---|---|---|
| **Betrachtung verschiedener TCEP Konzentrationen bei gleicher ß-Thiodiglycol Menge** | | | | | | | | | |
| | **frisch** | | | **7d/2-8°C** | | | **7d/37°C** | | |
| **Reagenzlot** | **Level 1** | **Level 2** | **ΔmE** | **Level 1** | **Level 2** | **AmE** | **Level 1** | **Level 2** | **ΔmE** |
| 10 g/L ß-Thiodiglycol ohne TCEP | 28,3 | 66,0 | 37,8 | 25,2 | 60,4 | 35,2 | 18,7 | 48,1 | 29,4 |
| 10 g/L ß-Thiodiglycol 0,1 mmol/L TCEP | 26,9 | 66,1 | 39,2 | 23,7 | 56,5 | 32,8 | 22,5 | 56,2 | 33,7 |
| 10 g/L ß-Thiodiglycol 0,2 mmol/L TCEP | 26,4 | 64,5 | 38,1 | 22,1 | 52,3 | 30,3 | 21,8 | 54,8 | 33,0 |
| 10 g/L ß-Thiodiglycol 0,3 mmol/L TCEP | 25,9 | 64,3 | 38,4 | 19,6 | 48,8 | 29,2 | 22,5 | 56,2 | 34,8 |
| 10 g/L ß-Thiodiglycol 0,4 mmol/L TCEP | 24,9 | 62,9 | 38,0 | 18,1 | 46,1 | 28,0 | 21,1 | 54,7 | 33,6 |
| 10 g/L ß-Thiodiglycol 0,5 mmol/L TCEP | 24,5 | 61,8 | 37,3 | 16,6 | 42,4 | 25,8 | 20,5 | 54,6 | 34,0 |

Evident ist der Signalabfall der HbA1c- Bestimmung bei 7d/ 2-8°C sowie bei 7d/ 37°C wenn nur Thiodiglycol eingesetzt wird. Die dargestellten Ergebnisse belegen dagegen klar den kombinatorischen Nutzen von TCEP und Thiodiglycol bei unterschiedlichen Konzentrationen TCEP.

## Patentansprüche

1. Verfahren zur Bestimmung der Menge an glykiertem Hämoglobin (HbA1c) in einer Probe, wobei man die folgenden Verfahrensschritte ausführt:
a) Hämolyse der Erythrocyten in der Probe zur Freisetzung des darin enthaltenen HbA1c,
b) Inkontaktbringen des in Verfahrensschritt a) freigesetzten HbA1c mit einem proteolytischen Agens zur Erzeugung von glykierten Hämoglobinabbauprodukten,
c) Bestimmung der Menge an HbA1c durch Quantifizierung der in Verfahrensschritt b) erzeugten glykierten Hämoglobinabbauprodukte,
**dadurch gekennzeichnet, dass** die Hämolyse in Verfahrensschritt a) durch Zugeben einer Hämolyselösung (H) erfolgt, wobei diese Hämolyselösung einen pH-Wert in dem Bereich von 1 bis 3 aufweist und ein hämolytisches Detergenz und wenigstens einen Stabilisator enthält, wobei der wenigstens eine Stabilisator ein 2-(Methacryloyloxyethyl)-2'-(trimethyl-ammoniumethyl)phosphatpolymer oder -copolymer mit der allgemeinen Formel (II) 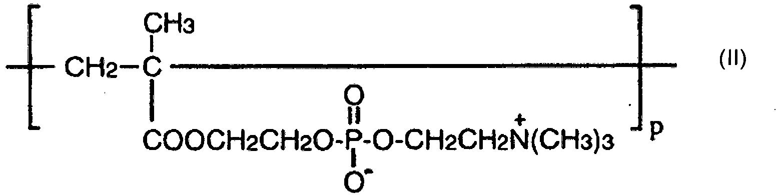 ist, wobei das Copolymer neben den Einheiten mit der oben angegebenen allgemeinen Formel (II) weitere Methacrylat-Einheiten umfasst, die mit aliphatischen oder aromatischen Resten verestert sind.

2. Verfahren nach Anspruch 1, **dadurch gekennzeichnet, dass** die Hämolyselösung wenigstens einen weiteren Stabilisator enthält, der ein Phosphatidylcholin mit der allgemeinen Formel (I) 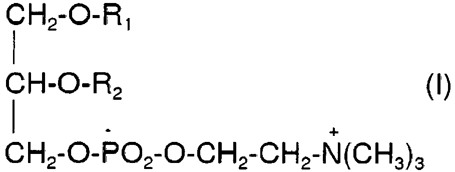 ist, wobei R₁ und R₂ ausgewählt sind unter vollständig gesättigten oder einfach oder mehrfach ungesättigten geradekettigen oder verzweigtkettigen Fettsäureresten mit einer Kettenlänge in dem Bereich von C8 bis C22.

3. Verfahren nach einem der Ansprüche 1 oder 2, **dadurch gekennzeichnet, dass** die Hämolyselösung wenigstens einen weiteren Stabilisator enthält, der ein zwitterionisches Detergenz mit der allgemeinen Formel (III) 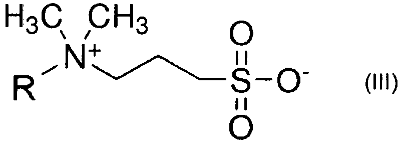 ist, wobei R ausgewählt ist unter einem Alkylrest mit einer Kettenlänge in dem Bereich von C8 bis C20.

4. Verfahren nach einem der Ansprüche 1 bis 3, **dadurch gekennzeichnet, dass** der erfindungsgemäß eingesetzte Stabilisator ein Gemisch von 2 oder mehr chemischen Verbindungen der vorgenannten Art ist.

5. Verfahren nach einem der Ansprüche 1 bis 4, **dadurch gekennzeichnet, dass** der erfindungsgemäß eingesetzte Stabilisator mit einer Konzentration im Bereich von 0,1 - 50g/L Hämolyselösung eingesetzt wird.

6. Verfahren nach einem der Ansprüche 1 bis 5, **dadurch gekennzeichnet, dass** die Quantifizierung der glykierten Hämoglobinabbauprodukte in Verfahrensschritt c) durch deren Oxidation mit Hilfe von Fructosylpeptidoxidase oder Fructosylaminosäureoxidase unter Erzeugung von Wasserstoffperoxid und durch Bestimmung der dabei entstehenden Menge an Wasserstoffperoxid erfolgt, wobei die Menge an Wasserstoffperoxid anhand der Farbreaktion eines Leucofarbstoffs in Gegenwart einer Peroxidase quantifiziert wird, und wobei der Leucofarbstoff in einer Lösung bereitgestellt wird, die zur Stabilisierung des Farbstoffes eine Verbindung der allgemeinen Formel (IV) enthält, 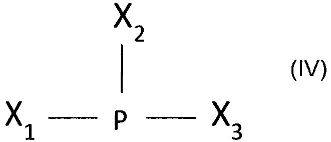 wobei P für ein Phosphoratom steht und wobei X₁, X₂ und X₃ unabhängig voneinander ausgewählt sind unter substituierten oder unsubstituierten gerade- oder verzweigtkettigen C₁-C₈-Alkylresten, substituierten oder unsubstituierten Cyclohexylresten und substituierten oder unsubstituierten Phenylresten.

7. Verfahren nach Anspruch 6, **dadurch gekennzeichnet, dass** die den Leucofarbstoff enthaltende Lösung zur Stabilisierung des Farbstoffes wenigstens eine Thioverbindung enthält.

8. Verfahren nach Anspruch 7, **dadurch gekennzeichnet, dass** die wenigstens eine Thioverbindung ausgewählt ist unter Thiodiglycol, Thioäpfelsäure, Thionicotinamid, Thio-NAD und Gemischen davon.

9. Verfahren nach einem der Ansprüche 1 bis 8, **dadurch gekennzeichnet, dass** man während oder zwischen den Verfahrensschritten a) - c) eine Bestimmung der Gesamthämoglobinkonzenztration durchführt.

10. Reagenz-Kit zur Anwendung in einem Verfahren zur Bestimmung der Menge an glykiertem Hämoglobin (HbA1c) in einer Probe, **dadurch gekennzeichnet, dass** das Reagenz-Kit wenigstens die folgenden Lösungen in separaten Gebinden umfasst:
- eine Hämolyselösung (H), enthaltend ein hämolytisch wirkendes Detergenz und 100 bis 5000 µmol/L eines zweiwertigen Metallions enthält, dass ausgewählt ist unter Fe²⁺, Mn²⁺, Co²⁺ und Zn²⁺,
- eine erste Reagenzienlösung (R1), enthaltend Fructosylpeptidoxidase oder Fructosylaminosäureoxidase und Peroxidase, und
- eine zweite Reagenzienlösung (R2), zusätzlich enthaltend eine Protease und pro 1000 kU/l an Protease jeweils 0,1 bis 2 mmol/l eines Chelators für zweiwertige Metallionen und 0,5 bis 10 mmol/l Ca²⁺ oder 0,5 bis 10 mmol/l Mg²⁺ enthält, wobei das molare Verhältnis Chelator:Ca²⁺ bzw. Chelator:Mg²⁺ in dem Bereich von 1:2 bis 1:20 liegt, und einen Leucofarbstoff,
wobei die Hämolyselösung (H) ein 2-(Methacryloyloxyethyl)-2'-(trimethyl-ammoniumethyl)phosphatpolymer oder-copolymer der allgemeinen Formel (II) gemäß Anspruch 1 enthält und wahlweise zusätzlich ein Phosphatidylcholin der allgemeinen Formel (I) gemäß Anspruch 2, ein zwitterionisches Detergenz der allgemeinen Formel (III) gemäß Anspruch 3 oder Gemische hiervon enthält und einen pH-Wert in dem Bereich von 1 bis 3 aufweist.

11. Reagenz-Kit nach Anspruch 10, **dadurch gekennzeichnet, dass** die Lösung (R2) zur Stabilisierung des Leucofarbstoffs wenigstens eine Verbindung der allgemeinen Formel (IV) enthält, 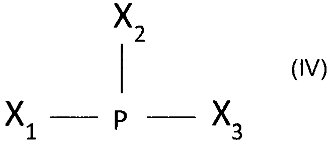 wobei P für ein Phosphoratom steht und wobei X₁, X₂ und X₃ unabhängig voneinander ausgewählt sind unter substituierten oder unsubstituierten gerade- oder verzweigtkettigen C₁-C₈-Alkylresten, substituierten oder unsubstituierten Cyclohexylresten und substituierten oder unsubstituierten Phenylresten.

12. Reagenz-Kit nach einem der Ansprüche 10 und 11, **dadurch gekennzeichnet, dass** die Lösung (R2) zur Stabilisierung des Leucofarbstoffs wenigstens eine Thioverbindung enthält, wobei die wenigstens eine Thioverbindung vorzugsweise ausgewählt ist unter Thiodiglycol, Thioäpfelsäure, Thionicotinamid, Thio-NAD und Gemischen davon.

13. Reagenz-Kit nach einem der Ansprüche 10 bis 12, **dadurch gekennzeichnet, dass** es in einem Verfahren nach einem der Ansprüche 1 bis 9 verwendet wird.

## Claims

1. A method of determining the amount of glycated haemoglobin (HbA1c) in a sample, wherein the following method steps are performed:
a) haemolysis of the erythrocytes in the sample to release the HbA1c contained therein,
b) bringing the HbA1c released in method step a) into contact with a proteolytic agent for producing glycated haemoglobin degradation products,
c) determining the amount of HbA1c by quantification of the glycated haemoglobin degradation products produced in method step b)
**characterised in that** haemolysis in method step a) is effected by adding a haemolysis solution (H), wherein that haemolysis solution has a pH-value in the range of 1 to 3 and contains a haemolytic detergent and at least one stabiliser, wherein the at least one stabiliser is a 2-(methacryloxyethyl)-2'-(trimethyl ammoniumethyl)phosphate polymer or copolymer of the general formula (II) 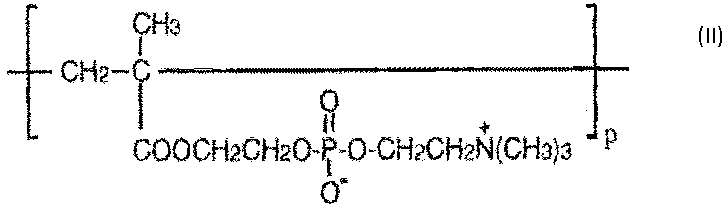 wherein in addition to the units of the above-specified general formula (II) the copolymer comprises further methacrylate units which are esterified with aliphatic or aromatic residues.

2. A method according to claim 1 **characterised in that** the haemolysis solution contains at least one further stabiliser which is a phosphatidylcholine of the general formula (I) 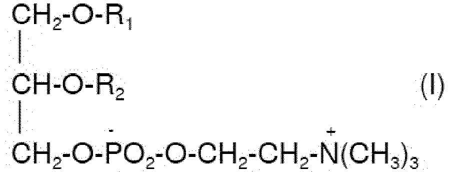 wherein R₁ and R₂ are selected from completely unsaturated or singly or multiply unsaturated straight-chain or branched-chain fatty acid residues of a chain length in the range of C8 to C22.

3. A method according to one of claims 1 or 2 **characterised in that** the haemolysis solution contains at least one further stabiliser which is a zwitterionic detergent of the general formula (III) 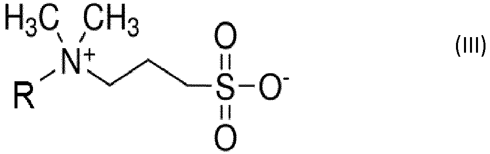 wherein R is selected from an alkyl residue of a chain length in the range of C8 to C20.

4. A method according to one of claims 1 to 3 **characterised in that** the stabiliser used according to the invention is a mixture of two or more chemical compounds of the above-indicated kind.

5. A method according to one of claims 1 to 4 **characterised in that** the stabiliser used according to the invention is used with a concentration in the range of 0.1 to 50 g/L haemolysis solution.

6. A method according to one of claims 1 to 5 **characterised in that** quantification of the glycated haemoglobin degradation products is effected in method step c) by oxidation thereof by means of fructosyl peptide oxidase or fructosyl amino acid oxidase with the production of hydrogen peroxide and by determining the resulting amount of hydrogen peroxide, wherein the amount of hydrogen peroxide is quantified on the basis of the colour reaction of a leuco dye in the presence of a peroxidase, and wherein the leuco dye is produced in a solution which for stabilisation of the dye contains a compound of the general formula (IV): 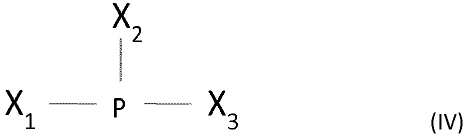 wherein P stands for a phosphorus atom and wherein X₁, X₂ and X₃ are selected independently of each other from substituted or unsubstituted straight-chain or branched-chain C₁C₈-alkyl residues, substituted or unsubstituted cyclohexyl residues and substituted or unsubstituted phenyl residues.

7. A method according to claim 6 **characterised in that** the solution containing the leuco dye for stabilisation of the dye contains at least one thio compound.

8. A method according to claim 7 **characterised in that** the at least one thio compound is selected from thiodiglycol, thiomalic acid, thionicontinomide, thio-NAD and mixtures thereof.

9. A method according to one of claims 1 to 8 **characterised in that** an operation for determining the total haemoglobin concentration is carried out during or between method steps a) - c).

10. A reagent kit for use in a method of determining the amount of glycated haemoglobin (HbA1c) in a sample, **characterised in that** the reagent kit includes at least the following solutions in separate containers:
- a haemolysis solution (H) containing a haemolytically acting detergent and 100 to 5000 µmol/L of a divalent metal ion selected from Fe²⁺, Mn²⁺, Co²⁺ and Zn²⁺,
- a first reagent solution (R1) containing fructosyl peptide oxidase or fructosyl amino acid oxidase and peroxidase, and
- a second reagent solution (R2) additionally containing a protease and per 1000 kU/l of protease respectively 0.1 to 2 mmol/l of a chelator for divalent metal ions and 0.5 to 10 mmol/l of Ca²⁺ or 0.5 to 10 mmol/l of Mg²⁺, wherein the molar ratio of chelator: Ca²⁺ and chelator: Mg²⁺ is in the range of 1:2 to 1:20, and a leuco dye
wherein the haemolysis solution (H) includes a 2-(methacryloyloxyethyl)-2'-(trimethyl ammoniumethyl)phosphate polymer or copolymer of the general formula (II) according to claim 1 and optionally a phosphatidylcholine of the general formula (I) according to claim 2, a zwitterionic detergent of the general formula (III) according to claim 3 or mixtures thereof, in addition, and has a pH-value in the range of 1 to 3.

11. A reagent kit according to claim 10 **characterised in that** the solution (R2) for stabilisation of the leuco dye contains at least one compound of the general formula (IV): 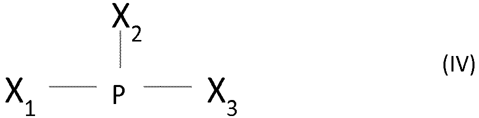 wherein P stands for a phosphorus atom and wherein X₁, X₂ and X₃ are selected independently of each other from substituted or unsubstituted straight-chain or branched-chain C₁C₈-alkyl residues, substituted or unsubstituted cyclohexyl residues and substituted or unsubstituted phenyl residues.

12. A reagent kit according to one of claims 10 and 11 **characterised in that** the solution (R2) for stabilising the leuco dye contains at least one thio compound, wherein the at least one thio compound is preferably selected from thiodiglycol, thiomalic acid, thionicotinamide, thio-NAD and mixtures thereof.

13. A reagent kit according to one of claims 10 to 12 **characterised in that** it is used in a method according to one of claims 1 to 9.

## Revendications

1. Procédé de détermination de la quantité d'hémoglobine glyquée (HbA1c) dans un échantillon, dans lequel on met en oeuvre les étapes suivantes :
a) hémolyse des érythrocytes se trouvant dans l'échantillon, pour libérer l'HbA1c qui y est contenue,
b) mise en contact de l'HbA1c libérée dans l'étape de procédé a) avec un agent protéolytique, pour générer des produits de dégradation de l'hémoglobine glyquée,
c) détermination de la quantité d'HbA1c par quantification des produits de dégradation de l'hémoglobine glyquée produits dans l'étape de procédé b),
**caractérisé en ce que** l'hémolyse a lieu dans l'étape de procédé a) par addition d'une solution d'hémolyse (H), cette solution d'hémolyse présentant une valeur de pH comprise dans la plage de 1 à 3, et contenant un détergent pour hémolyse et au moins un stabilisant, le stabilisant, au moins au nombre de un, étant un polymère ou un copolymère de phosphate de 2-(méthacryloyloxyéthyl)-2'-(triméthylammoniuméthyle), ayant la formule générale (II) 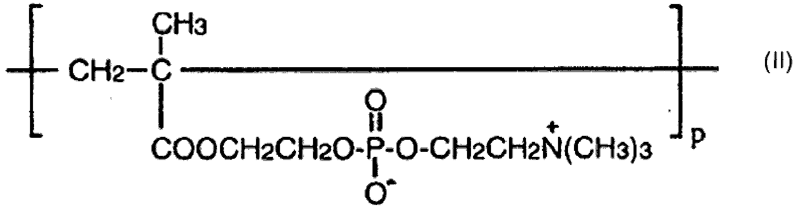 le copolymère comprenant, outre les motifs ayant la formule générale (II) indiquée ci-dessus, des motifs méthacrylate supplémentaires qui sont estérifiés avec des radicaux aliphatiques ou aromatiques.

2. Procédé selon la revendication 1, **caractérisé en ce que** la solution d'hémolyse contient au moins un stabilisant supplémentaire qui est une phosphatidylcholine de formule générale (I) 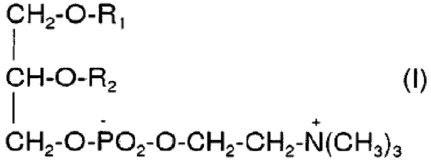 dans laquelle Ri et R₂ sont choisis parmi des résidus d'acides gras à chaîne droite ou ramifiée, entièrement saturés ou mono- ou polyinsaturés, présentant une longueur de chaîne comprise dans la plage de C8 à C22.

3. Procédé selon l'une des revendications 1 ou 2, **caractérisé en ce que** la solution d'hémolyse contient au moins un stabilisant supplémentaire qui est un détergent zwitterionique de formule générale (III) 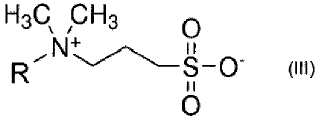 dans laquelle R est choisi parmi des radicaux alkyle ayant une longueur de chaîne comprise dans la plage de C8 à C20.

4. Procédé selon l'une des revendications 1 à 3, **caractérisé en ce que** le stabilisant utilisé selon l'invention est un mélange de 2 composés chimiques ou plus du type mentionné précédemment.

5. Procédé selon l'une des revendications 1 à 4, **caractérisé en ce que** le stabilisant utilisé selon l'invention est utilisé à une concentration comprise dans la plage de 0,1 à 50 g/L de solution d'hémolyse.

6. Procédé selon l'une des revendications 1 à 5, **caractérisé en ce que** la quantification des produits de dégradation de l'hémoglobine glyquée est, dans l'étape c), réalisée par leur oxydation à l'aide de fructosylpeptidoxydase ou de fructosyl-acide aminé-oxydase, avec production de peroxyde d'hydrogène, et par détermination de la quantité de peroxyde d'hydrogène qui se forme à cette occasion, la quantité de peroxyde d'hydrogène étant quantifiée à l'aide de la réaction colorée d'un leuco-dérivé de colorant en présence d'une peroxydase, et le leuco-dérivé de colorant étant fourni dans une solution qui, pour la stabilisation du colorant, contient un composé de formule générale (IV), 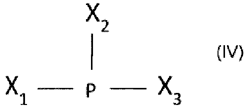 dans laquelle P représente un atome de phosphore, et X₁, X₂ et X₃ sont choisis indépendamment les uns des autres parmi des radicaux alkyle en Ci-C₈ à chaîne droite ou ramifiée, substitués ou non-substitués, des radicaux cyclohexyle substitués ou non-substitués, et des radicaux phényle substitués ou non-substitués.

7. Procédé selon la revendication 6, **caractérisé en ce que** la solution contenant le leuco-dérivé de colorant contient, pour la stabilisation du colorant, au moins un composé thio.

8. Procédé selon la revendication 7, **caractérisé en ce que** le composé thio, au moins au nombre de un, est choisi parmi le thiodiglycol, l'acide thiomalique, le thionicotinamide, le thio-NAD et des mélanges de ceux-ci.

9. Procédé selon l'une des revendications 1 à 8, **caractérisé en ce que**, pendant ou entre les étapes de procédé a) - c), on procède à une détermination de la concentration totale en hémoglobine.

10. Trousse de réactifs destinée à une utilisation dans un procédé destiné à déterminer la quantité d'hémoglobine glyquée (HbA1c) se trouvant dans un échantillon, **caractérisée en ce que** la trousse de réactifs contient au moins les solutions suivantes, dans des récipients distincts :
- une solution d'hémolyse (H) contenant un détergent à effet hémolytique et 100 à 5000 µmol/L d'un ion métallique divalent qui est choisi parmi Fe²⁺, Mn²⁺, Co²⁺ et Zn²⁺,
- une première solution de réactifs (R1) contenant de la fructosylpeptidoxydase ou de la fructosyl-acide aminé-oxydase et de la peroxydase, et
- une deuxième solution de réactifs (R2) contenant en outre une protéase et, pour 1000 kU/I de protéase, respectivement 0,1 à 2 mmol/l d'un chélatant pour ions métalliques divalents et 0,5 à 10 mmol/l de Ca²⁺ ou 0,5 à 10 mmol/l de Mg²⁺, le rapport molaire chélatant:Ca²⁺ ou chélatant:Mg²⁺ étant compris dans la plage de 1:2 à 1:20, et un leuco-dérivé de colorant,
la solution d'hémolyse (H) contenant un polymère ou un copolymère de phosphate de 2-(méthacryloyloxyéthyl)-2'-(triméthyl-ammoniuméthyle) de formule générale (II) selon la revendication 1, et contenant au choix en outre une phosphatidylcholine de formule générale (I) selon la revendication 2, un détergent zwitterionique de formule générale (III) selon la revendication 3 ou des mélanges de ceux-ci, et présentant une valeur de pH comprise dans la plage de 1 à 3.

11. Trousse de réactifs selon la revendication 10, **caractérisée en ce que** la solution (R2) destinée à la stabilisation du leuco-dérivé de colorant contient au moins un composé de formule générale (IV) 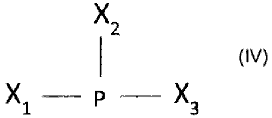 dans laquelle P représente un atome de phosphore, et X₁, X₂ et X₃ sont choisis indépendamment les uns des autres parmi des radicaux alkyle en Ci-C₈ à chaîne droite ou ramifiée, substitués ou non-substitués, des radicaux cyclohexyle substitués ou non-substitués, et des radicaux phényle substitués ou non-substitués.

12. Trousse de réactifs selon l'une des revendications 10 et 11, **caractérisée en ce que** la solution (R2) destinée à la stabilisation du leuco-dérivé de colorant contient au moins un composé thio, le composé thio, au moins au nombre de un, étant de préférence choisi parmi le thiodiglycol, l'acide thiomalique, le thionicotinamide, le thio-NAD et des mélanges de ceux-ci.

13. Trousse de réactifs selon l'une des revendications 10 à 12, **caractérisée en ce qu'**elle est utilisée dans un procédé selon l'une des revendications 1 à 9.
